# EUROPÄISCHE PATENTSCHRIFT

(11) **EP 0 325 129 B1**
(45) Veröffentlichungstag und Bekanntmachung des Hinweises auf die Patenterteilung: **07.09.1994**
(21) Anmeldenummer: 89100249.5
(22) Anmeldetag: 09.01.1989
(51) Int. Cl.: C07D 213/55, C07D 405/04, C07D 409/04, A61K 31/44

(54) **Disubstituierte Pyridine**
Disubstituted pyridines
Pyridines disubstituées

(30) Priorität: 20.01.1988 DE 3801440; 29.07.1988 IT 2158788
(43) Veröffentlichungstag der Anmeldung: 26.07.1989
(73) Patentinhaber: BAYER AG, 51368 Leverkusen (DE)
(72) Erfinder: Angerbauer, Rolf, Dr., D-5600 Wuppertal 1 (DE); Fey, Peter, Dr., D-5600 Wuppertal 1 (DE); Hübsch, Walter, Dr., D-5600 Wuppertal 1 (DE); Philipps, Thomas, Dr., D-5000 Koeln 80 (DE); Bischoff, Hilmar, Dr., D-5600 Wuppertal 2 (DE); Petzinna, Dieter, Dr. Dr., D-4000 Duesseldorf 21 (DE); Schmidt, Delf, Dr., D-5600 Wuppertal 1 (DE); Thomas, Günter, Dr., I-20020 Arese (Mi) (IT)

(56) Entgegenhaltungen:
- US-A- 4 681 893

## Beschreibung

Die Erfindung betrifft disubstituierte Pyridine, Zwischenverbindungen zu ihrer Herstellung, ihre Herstellung und ihre Verwendung in Arzneimitteln.

Es ist bekannt, daß aus Pilzkulturen isolierte Lactonderivate Inhibitoren der 3-Hydroxy-3-methyl-glutaryl Coenzym A Reduktase (HMG-CoA-Reduktase) sind [Mevinolin, EP-A 22 478; US 4 231 938]. Darüberhinaus sind auch bestimmte Indolderivate bzw. Pyrazolderivate Inhibitoren der HMG-CoA-Reduktase [EP-A 1 114 027; US 4 613 610].

Die US 4 681 893 offenbart 4-Hydroxypyran-2-on-pyrrol-derivate, wobei die Lactongruppierung bzw. das offenkettige Säureanaloge am Ringstickstoff gebunden ist, die als Inhibitoren der HMG-CoA-Reduktase eingesetzt werden können.

Es wurden nun disubstituierte Pyridine der allgemeinen Formeln (Ia,b) 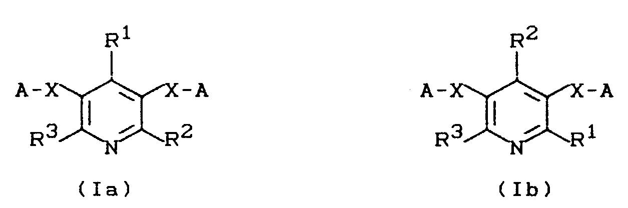
in welcher
- R¹: - Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder verschieden substituiert sein kann durch Fluor, Chlor, Brom, C₁-C₆-Alkyl, C₁-C₆-Alkoxy, Phenyl, Phenoxy, Trifluormethyl, Trifluormethoxy oder C₁-C₆-Alkoxycarbonyl, oder
- Phenyl oder Naphthyl bedeutet, das bis zu 4-fach gleich oder verschieden substituiert sein kann durch C₁-C₆-Alkyl, C₁-C₆-Alkoxy, C₁-C₆-Alkylthio, C₁-C₆-Alkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, C₁-C₆-Alkoxycarbonyl oder durch eine Gruppe der Formel -NR⁴R⁵,
wobei
- R⁴ und R⁵: gleich oder verschieden sind und C₁-C₆-Alkyl, Phenyl, Benzyl, Acetyl, Benzoyl, Phenylsulfonyl oder C₁-C₆-Alkylsulfonyl bedeuten,
- R²: - Cyclopropyl, Cyclopentyl oder Cyclohexyl bedeutet, oder
- C₁-C₆-Alkyl bedeutet, das substituiert sein kann durch Fluor, Chlor, Brom, Cyano, C₁-C₆-Alkoxy, C₁-C₆-Alkylthio, C₁-C₆-Alkylsulfonyl, Phenyl, Trifluormethyl, Trifluormethoxy, Trifluormethylsulfonyl, C₁-C₆-Alkoxycarbonyl, Benzoyl, C₁-C₆-Alkylcarbonyl, durch eine Gruppe der Formel -NR⁴R⁵,
worin
- R⁴ und R⁵: die oben angegebene Bedeutung haben, oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, C₁-C₆-Alkyl, C₁-C₆-Alkoxy, Trifluormethyl, oder Trifluormethoxy substituiert sein können,
- R³: - Wasserstoff, oder
- Cyclopropyl, Cyclopentyl oder Cyclohexyl bedeutet, oder
- C₁-C₆-Alkyl bedeutet, das substituiert sein kann durch Fluor, Chlor, Brom, Cyano, C₁-C₆-Alkoxy, C₁-C₆-Alkylthio, C₁-C₆-Alkylsulfonyl, Trifluormethyl, Trifluormethoxy, Trifluormethylsulfonyl, C₁-C₆-Alkoxycarbonyl, Benzoyl, C₁-C₆-Alkylcarbonyl, durch eine Gruppe der Formel -NR₄R₅,
worin
- R⁵ und R⁶: die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl-und Arylreste bis zu 2-fach gleich oder verschieden durch Fluor, Chlor, Brom, C₁-C₆-Alkyl, C₁-C₆-Alkoxy, Trifluormethyl, oder Trifluormethoxy substituiert sein können,
- Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder verschieden substituiert sein kann durch Fluor, Chlor, Brom, C₁-C₆-Alkyl, C₁-C₆-Alkoxy, Phenyl, Phenoxy, Trifluormethyl, Trifluormethoxy oder C₁-C₆-Alkoxycarbonyl, oder
- Phenyl oder Naphthyl bedeutet, das bis zu 4-fach gleich oder verschieden substituiert sein kann durch C₁-C₆-Alkyl, C₁-C₆-Alkoxy, C₁-C₆-Alkylthio, C₁-C₆-Alkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, C₁-C₆-Alkoxycarbonyl oder durch eine Gruppe der Formel -NR⁵R⁶,
wobei
- R⁵ und R⁶: die oben angegebene Bedeutung haben,
- X: - eine Gruppe der Formel -CH=CH- bedeutet
und
- A: - eine Gruppe der Formel 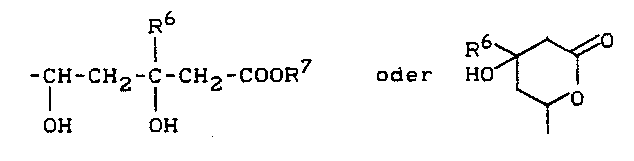
bedeutet,
worin
- R⁶: - Wasserstoff oder C₁-C₆-Alkyl bedeutet,
und
- R⁷: - C₁-C₆-Alkyl, Phenyl oder Benzyl bedeutet, oder
- ein physiologisch verträgliches Kation bedeutet.
gefunden.

Überraschenderweise zeigen die erfindungsgemäßen disubstituierten Pyridine eine überlegene inhibitorische Wirkung auf die HMG-CoA Reduktase (3-Hydroxy-3-methylglutaryl Coenzym A Reduktase).

Bevorzugt sind Verbindungen der allgemeinen Formel (Ia) und (Ib)
in welcher
- R¹: - Pyridyl, Pyrimidyl, Chinolyl oder Isochinolyl bedeutet, das durch Fluor, Chlor, Methyl, Methoxy oder Trifluormethyl substituiert sein kann, oder
- Phenyl bedeutet, das bis zu 3-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl,
- R²: - Cyclopropyl, Cyclopentyl oder Cyclohexyl bedeutet, oder
- Methyl, Ethyl, Propyl, Isopropyl, Butyl, sec.-Butyl oder tert.Butyl bedeutet, das substituiert sein kann durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,
- R³: - Wasserstoff, Cyclopropyl, Cyclopentyl oder Cyclohexyl bedeutet, oder
- Methyl, Ethyl, Propyl, Isopropyl, Butyl, Isobutyl, tert.Butyl, Pentyl, Isopentyl, Hexyl oder Isohexyl bedeutet, das substituiert sein kann durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe -NR⁴R⁵,
   wobei
- R⁴ und R⁵: gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,
oder durch Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy Substituiert sein können, oder
- Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl bedeutet, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder
- Phenyl bedeutet, das bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Bensylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe -NR⁴R⁵ substituiert sein kann,
wobei
- R⁴ und R⁵: die oben angegebene Bedeutung haben,
- X: - eine Gruppe der Formel -CH=CH- bedeutet
und
- A: - eine Gruppe der Formel 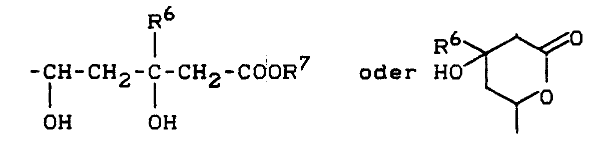 bedeutet,
worin
- R⁶: - Wasserstoff, Methyl, Ethyl, Propyl, Isopropyl, Butyl, Isobutyl oder tert.Butyl bedeutet
und
- R⁷: - Wasserstoff, Methyl, Ethyl, Propyl, Isopropyl, Butyl, Isobutyl, tert.Butyl oder Benzyl bedeutet, oder
- ein Natrium-, Kalium-, Calcium- oder Magnesium- oder Ammoniumion bedeutet.

Besonders bevorzugt sind Verbindungen der allgemeinen Formeln (Ia) und (Ib)
in welcher
- R¹: - Phenyl bedeutet, das bis zu 2-fach gleich oder verschieden durch Methyl, Ethyl, Propyl, Isopropyl, Phenoxy und/oder Fluor substituiert sein kann,
- R²: - für Cyclopropyl oder Cyclohexyl steht oder Methyl, Ethyl, Propyl, Isopropyl, Butyl, Isobutyl oder tert.Butyl bedeutet, das durch Fluor, Chlor, Methoxy, Phenyl oder Phenoxy substituiert sein kann,
- R³: - Wasserstoff, Cyclopropyl, Cyclopentyl oder Cyclohexyl oder Phenyl bedeutet, oder
- Methyl, Ethyl, Propyl, Isopropyl, Butyl oder Benzyl bedeutet,
- X: - eine Gruppe der Formel 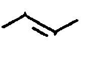 (E-konfiguriert) bedeutet
und
- A: - eine Gruppe der Formel 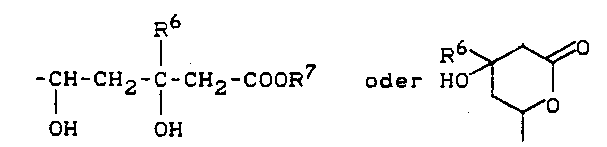 bedeutet,
worin
- R⁶: - Wasserstoff bedeutet
und
- R⁷: - Wasserstoff, Methyl oder Ethyl bedeutet, oder
- ein Natrium- oder Kaliumkation bedeutet.

Die erfindungsgemäßen disubstituierten Pyridine der allgemeinen Formel (Ia,b) haben mehrere asymmetrische Kohlenstoffatome und können daher in verschiedenen stereochemischen Formen existieren. Sowohl die einzelnen Isomeren als auch deren Mischungen sind Gegenstand der Erfindung.

Je nach der Bedeutung der Gruppen X bzw. der Reste A ergeben sich unterschiedliche Stereoisomere, die im folgenden näher erläutert werden sollten:
a) Steht die Gruppe -X- für eine Gruppe der Formel - CH=CH- , so können die erfindungsgemäßen Verbindungen in zwei stereoisomeren Formen existieren, die an der Doppelbindung E-konfiguriert oder Z-konfiguriert sein können,
   Bevorzugt sind diejenigen Verbindungen der allgemeinen Formeln (Ia,b), die E-konfiguriert sind.
b) Steht der Rest -A für eine Gruppe der Formel 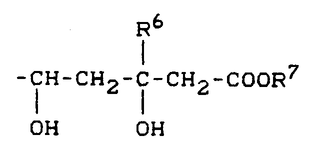 so besitzen die Verbindungen der allgemeinen Formeln mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration oder in der threo-Konfiguration vorliegen.

Sowohl von den Verbindungen in Erythro- als auch in Threo-Konfiguration existieren wiederum jeweils zwei Enantiomere, nämlich 3R,5S-Isomeres bzw. 3S,5R-Isomeres (Erythro-Form) sowie 3R,5R-Isomeres und 3S,5S-Isomeres (Threo-Form).

Bevorzugt sind hierbei die Erythro-konfigurierten Isomeren, besonders bevorzugt das 3R,5S-Isomere sowie das 3R,5S-3S,5R-Racemat.
c) Steht der Rest -A für eine Gruppe der Formel 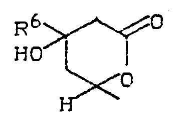 so besitzen die disubstituierten Pyridine mindestens zwei asymmetrische Kohlenstoffatome, nämlich das Kohlenstoffatom an dem die Hydroxygruppe gebunden ist, und das Kohlenstoffatom an welchem der Rest der Formel 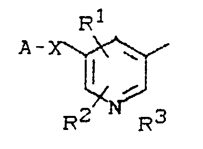 gebunden ist. Je nach der Stellung der Hydroxygruppe zur freien Valenz am Lactonring können die disubstituierten Pyridine als cis-Lactone oder als trans-Lactone vorliegen.

Sowohl vom cis-Lacton aus als auch vom trans-Lacton existieren wiederum jeweils zwei Isomere nämlich das 4R,6R-Isomere bzw. das 4S,6S-Isomere (cis-Lacton), sowie das 4R,6S-Isomere bzw. 4S,6R-Isomere (trans-Lacton). Bevorzugte Isomere sind die trans-Lactone. Besonders bevorzugt ist hierbei das 4R,6S-Isomere (trans) sowie das 4R,6S-4S,6R-Racemat.

Darüber hinaus ergeben sich weitere Möglichkeiten der Isomerenbildung, da die erfindungsgemäßen disubstituierten Pyridine durch zwei Gruppen der Formel -X-A substituiert sind, Auch für die zweite Gruppe -X-A im Molekül gilt das oben gesagte. Es sind ebenso alle Stereoisomeren Gegenstand der Erfindung, die durch die zweite Gruppe der Formel -X-A, insbesondere im Zusammenhang mit der ersten Gruppe -X-A entstehen.

Außerdem wurde ein Verfahren zur Herstellung der disubstituierten Pyridine der allgemeinen Formel (Ia,b) 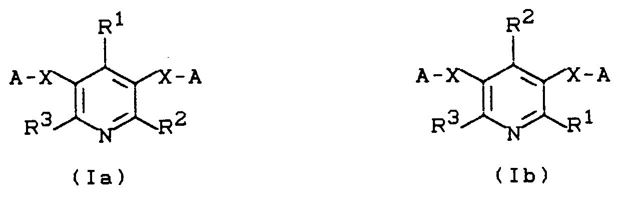
in welcher
R¹, R², R³, X und A die oben angegebene Bedeutung haben,
gefunden,
das dadurch gekennzeichnet ist, daß man
Ketone der allgemeinen Formel (VIII) 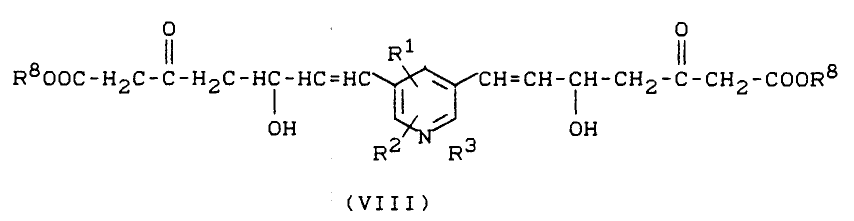
in welcher
R¹, R² und R³ die oben angegebene Bedeutung haben,
und
- R⁸: - für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
und gegebenenfalls Isomeren trennt.

Das erfindungsgemäße Verfahren kann durch das folgendes Formelschema erläutert werden: 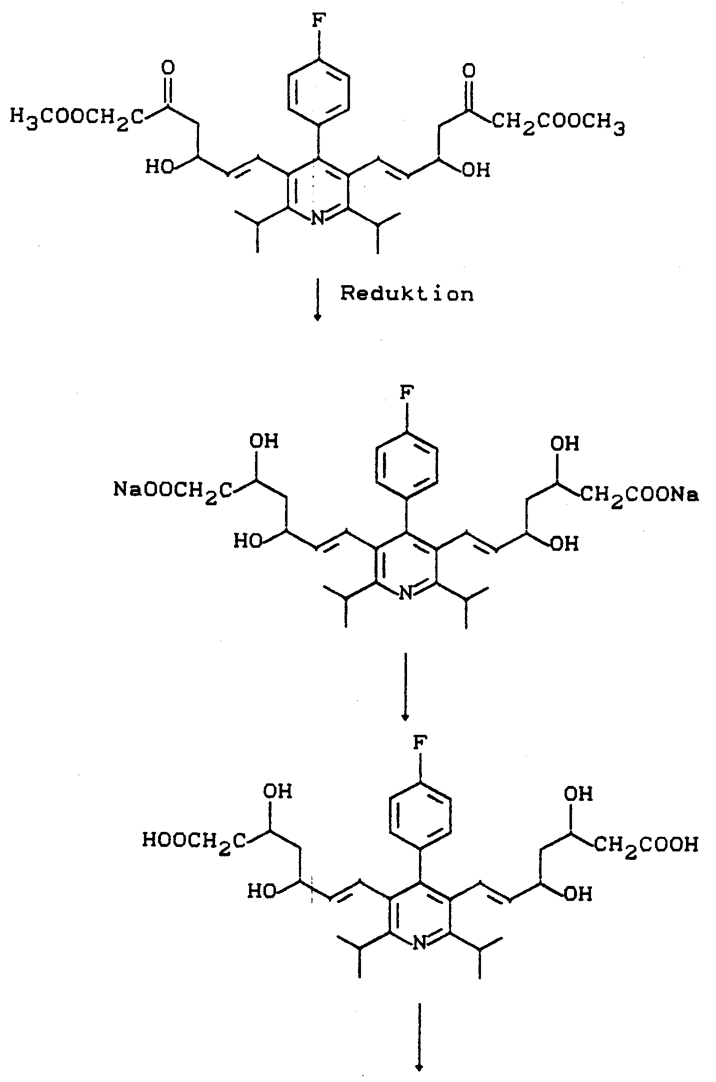 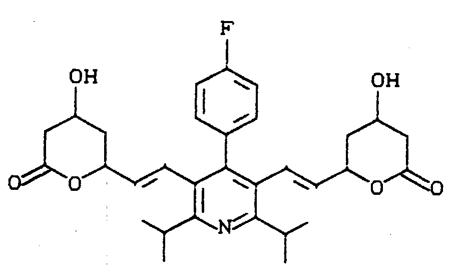
Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkyl-hydrido-borate, Natrium-trialkyl-hydrido-boranaten, Natrium-cyano-trihydrido-borat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern, Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispielsweise die Alkoxycarbonylgruppe nicht verändert werden. Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -80°C bis +30°C, bevorzugt von -78°C bis 0°C.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt. 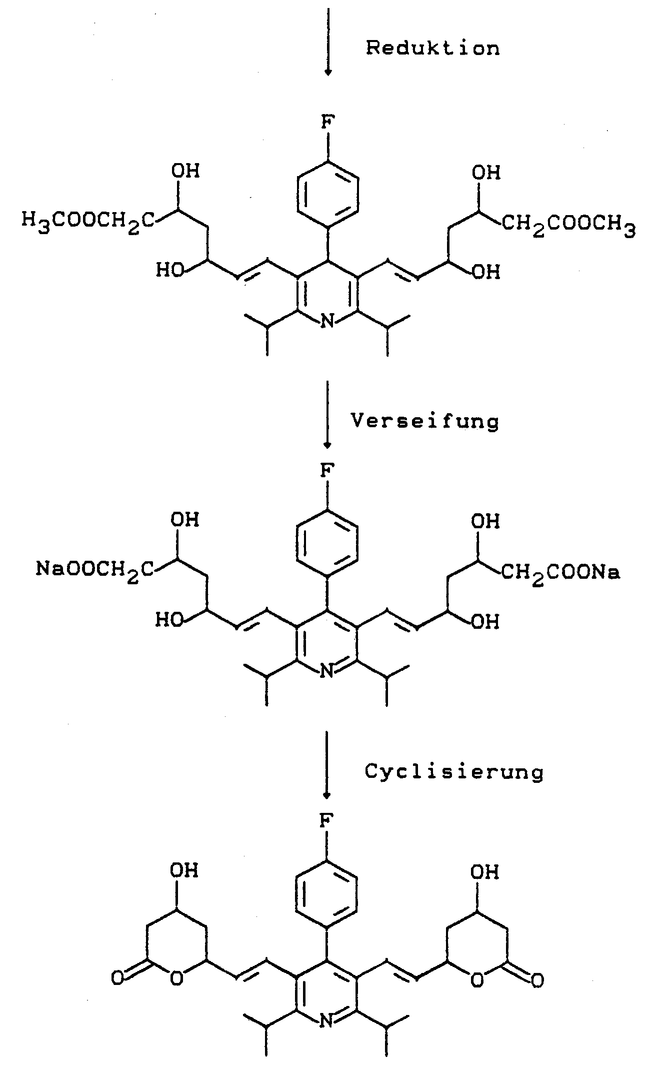
Darüber hinaus ist es auch möglich, die Reduktion der Carbonylgruppe und die Reduktion der Doppelbindung in zwei getrennten Schritten durchzuführen.

Die Carbonsäuren im Rahmen der allgemeinen Formel entsprechen der Formel (Ic) 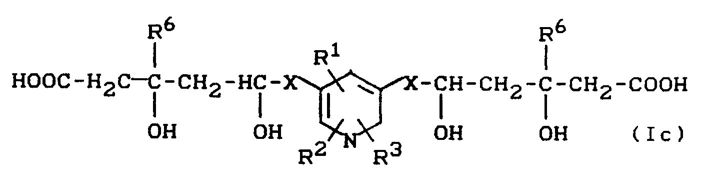
in welcher
R¹, R², R³, R⁶ und X die oben angegebene Bedeutung haben.

Die Carbonsäureester im Rahmen der allgemeinen Formel entsprechen der Formel (Id) 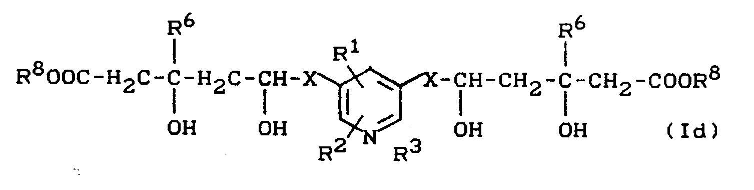
in welcher
R¹, R², R³, R⁶ und X die oben angegebene Bedeutung haben,
und
- R⁸: - für Alkyl steht.

Die Salze der erfindungsgemäßen Verbindungen im Rahmen der allgemeinen Formel entsprechen der Formel (Ie) 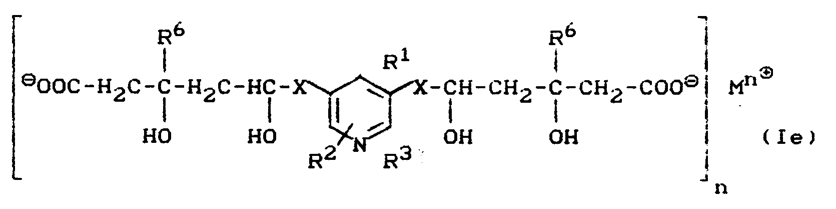
in welcher
R¹, R², R³, R⁶ und X die oben angegebene Bedeutung haben,
und 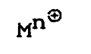
für ein Kation steht.

Die Lactone im Rahmen der allgemeinen Formel entsprechen der Formel (If) 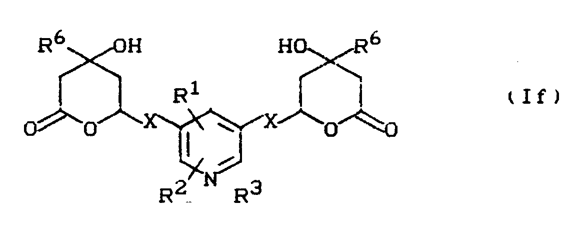
in welcher
R¹, R², R³, R⁶ und X die oben angegebene Bedeutung haben.

Zur Herstellung der erfindungsgemäßen Carbonsäuren der allgemeinen Formel (Ic) werden im allgemeinen die Carbonsäureester der allgemeinen Formel (Id) oder die Lactone der allgemeinen Formel (If) nach üblichen Methoden verseift, Die Verseifung erfolgt im allgemeinen indem man die Ester oder die Lactone in inerten Lösemitteln mit üblichen Basen behandelt, wobei im allgemeinen zunächst die Salze der allgemeinen Formel (Ie) entstehen, die anschließend in einem zweiten Schritt durch Behandeln mit Säure in die freien Säuren der allgemeinen Formel (Ic) überführt werden können.

Als Basen eignen sich für die Verseifung die üblichen anorganischen Basen, Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen (Ie) als Zwischenprodukte, die isoliert werden können. Die erfindungsgemäßen Säuren (Ic) erhält man durch Behandeln der Salze (Ie) mit üblichen anorganischen Säu ren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ic) hierbei als vorteilhaft erwiesen, die basische Reaktionsmischung der Verseifung in einem zweiten Schritt ohne Isolierung der Salze anzusäueren. Die Säuren können dann in üblicher Weise isoliert werden.

Zur Herstellung der erfindungsgemäßen Lactone der Formel (If) werden im allgemeinen die erfindungsgemäßen Carbonsäuren (Ic) nach üblichen Methoden cyclisiert, beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40°C bis +200°C, bevorzugt von -25°C bis +50°C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüberhinaus wird die Cyclisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbondiimide werden bevorzugt N,N'-Dicyclohexylcarbodiimid-Paratoluolsulfonat, N-Cyclohexyl-N'-[2-(N''-methylmorpholinium)ethyl]carbodiimid oder N-(3-Dimethylaminopropyl)-N'-ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen. Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen, Besonders bevorzugt werden Chlorkohlenwasserstoffe wie beispielsweise Methylenchlorid, Chloroform oder Tetrachlorkohlenstoff eingesetzt.

Die Reaktion wird im allgemeinen in einem Temperaturbereich von 0°C bis +80°C, bevorzugt von +10°C bis +50°C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 beschrieben wird. Bevorzugt wird hierbei die Trennung der Isomeren auf der Stufe der racemischen Lactone. Besonders bevorzugt wird hierbei die racemische Mischung der trans-Lactone (VII) durch Behandeln entweder mit D-(+)- oder L-(-)-α-Methylbenzylamin nach üblichen Methoden in die diastereomeren Dihydroxyamide (Ig) 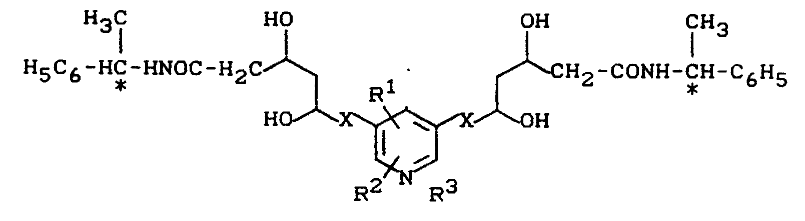
überführt, die anschließend wie üblich durch Chromatographie oder Kristallisation in die einzelnen Diastereomeren getrennt werden können. Anschließende Hydrolyse der reinen diastereomeren Amide nach üblichen Methoden, beispielsweise durch Behandeln der diastereomeren Amide mit anorganischen Basen wie Natriumhydroxid oder Kaliumhydroxid in Wasser und/oder organischen Lösemitteln wie Alkoholen z.B. Methanol, Ethanol, Propanol oder Isopropanol, ergeben die entsprechenden enantiomerenreinen Dihydroxysäuren (Ic), die wie oben beschrieben durch Cyclisierung in die enantiomerenreinen Lactone überführt werden können. Im allgemeinen gilt für die Herstellung der erfindungsgemäßen Verbindungen der allgemeinen Formel (Ia,b) in enantiomerenreiner Form, daß die Konfiguration der Endprodukt nach der oben beschriebenen Methode abhängig ist von der Konfiguration der Ausgangsstoffe.

Die Isomerentrennung soll im folgenden Schema beispielhaft erläutert werden: 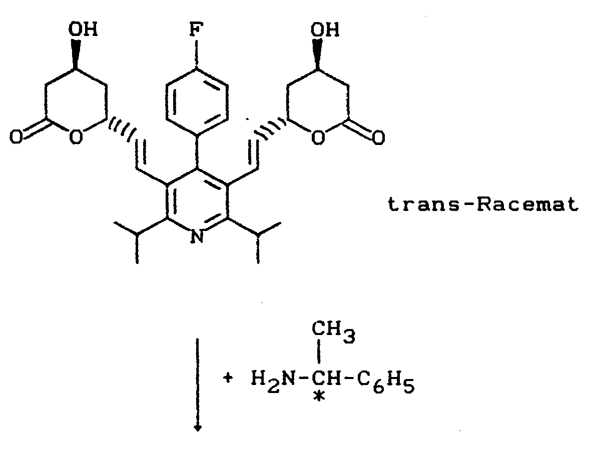 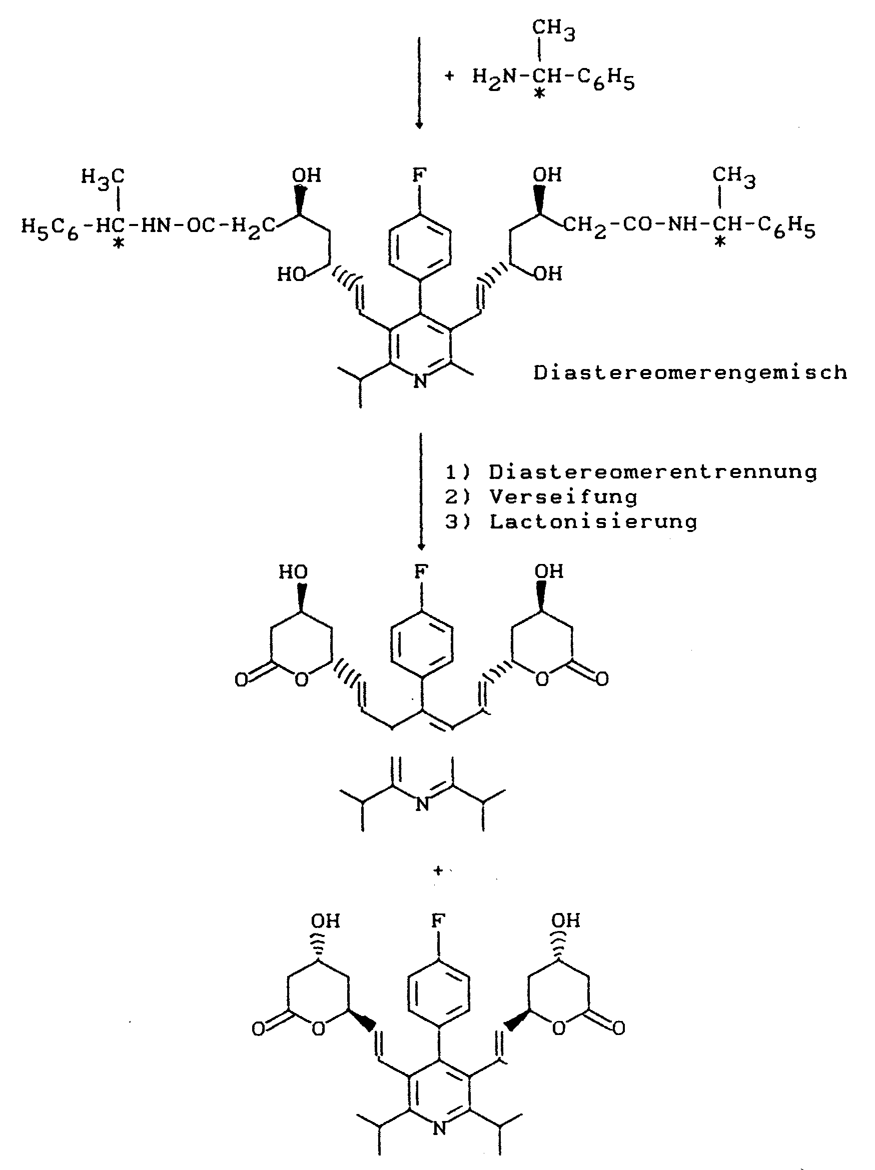
Die als Ausgangsstoffe eingesetzten Ketone (VIII) sind neu.

Es wurde ein Verfahren zur Herstellung der erfindungsgemäßen Ketone der allgemeinen Formel (VIII) 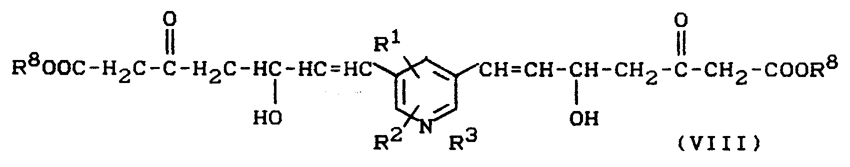
in welcher
R¹, R² und R³ die oben angegebene Bedeutung haben
und
- R⁸: - für Alkyl steht,
gefunden, das dadurch gekennzeichnet ist, daß man Aldehyde der allgemeinen Formel (IX) 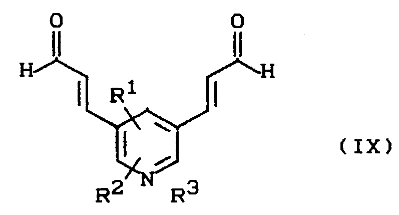
in welcher
R¹, R² und R³ die oben angegebene Bedeutung haben,
in inerten Lösemitteln mit Acetessigester der allgemeinen Formel (X) 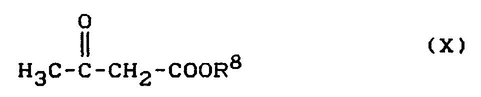
in welcher
R⁸ die oben angegebene Bedeutung hat,
in Anwesenheit von Basen umsetzt.

Das erfindungsgemäße Verfahren kann beispielsweise durch folgendes Formelschema erläutert werden: 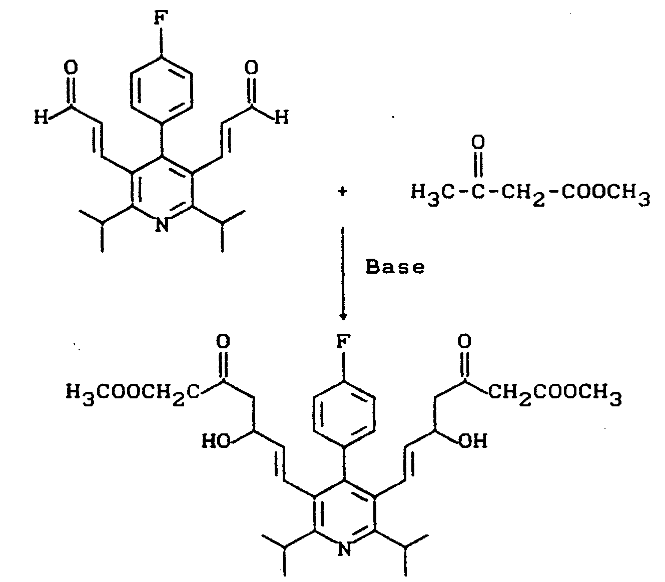
Als Basen kommen hierbei die üblichen stark basischen Verbindungen in Frage. Hierzu gehören bevorzugt lithiumorganische Verbindungen wie beispielsweise N-Butyllithium, sec.Butyllithium, tert.Butyllithium oder Phenyllithium, oder Amide wie beispielsweise Lithiumdiisopropylamid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden N-Butyllithium oder Natriumhydrid oder deren Gemisch eingesetzt.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80°C bis +50°C, bevorzugt von -20°C bis +30°C durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol, bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt: Acetessigsäuremethylester, Acetessigsäureethylester, Acetessigsäurepropylester, Acetessigsäureisopropylester.

Die als Ausgangsstoffe eingesetzten Aldehyde der allgemeinen Formel (IX) sind neu.

Die Herstellung der Aldehyde kann durch das folgende Reaktionsschema beispielhaft für die Verbindungen des Typs (Ia) dargestellt werden: 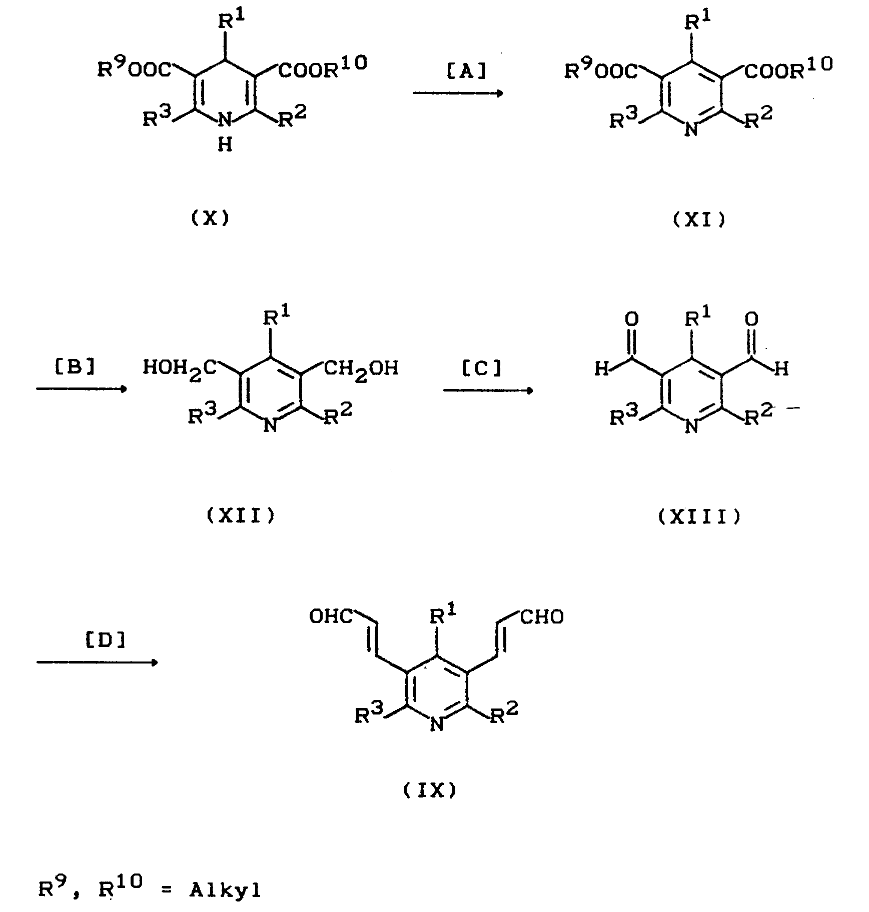
Hierbei werden im ersten Schritt [A] die Dihydropyridine der allgemeinen Formel (X) in geeigneten Lösemitteln, mit geeigneten Oxidationsmitteln, oxidiert. Bevorzugt werden die Dihydropyridine in Chlorkohlenwasserstoffen wie beispielsweise Methylenchlorid, mit 2,2-Dichlor-5,6-dicyan-p-benzochinon bei Raumtemperatur, oder mit Chromtrioxid in Eisessig bei erhöhten Temperaturen, bevorzugt unter Rückflußtemperatur, zu den Pyridinen der Formel (XI) oxidiert. Im zweiten Schritt [B] werden die Pyridine (XI) in inerten Lösemitteln wie Ethern, beispielsweise Diethylether, Dioxan oder Tetrahydrofuran, bevorzugt in Tetrahydrofuran, mit Metallhydriden wie Lithiumaluminiumhydrid, Natriumcyano-borhydrid, Diisobutylaluminiumhydrid oder Natrium-bis-(2-methoxyethoxy)-dihydroaluminat, in einem Temperaturbereich von -80°C bis +40°C, bevorzugt von -70°C bis Raumtemperatur zu den Hydroxyverbindungen der allgemeinen Formel (XII) reduziert. Im dritten Schritt [C] werden die Hydroxyverbindungen (XII) nach bekannten Methoden mit Oxidationsmitteln wie Pyridiniumchlorochromat, gegebenenfalls in Anwesenheit von Aluminiumoxid, in inerten Lösemitteln wie Chlorkohlenwasserstoffen, bevorzugt Methylenchlorid, bei Raumtemperatur oder mit Trifluoressigsäure und Dimethylsulfoxid (Swern-Oxidation) oder aber nach anderen für die Oxidation von Hydroxymethylverbindungen zu Aldehyden üblichen Methoden zu den Aldehyden (XIII) oxidiert. Im vierten Schritt [D] werden die Aldehyde (XIII) durch Umsetzen mit Diethyl-2-(cyclohexylamino)-vinyl-phosphonat in inerten Lösemitteln wie Ethern, bevorzugt in Tetrahydrofuran in Anwesenheit von Natriumhydrid, in einem Temperaturbereich von -20°C bis +30°C, bevorzugt von - 5°C bis Raumtemperatur in die Verbindungen (IX) überführt.

Die als Ausgangsstoffe eingesetzten Dihydropyridine der allgemeinen Formel (X) sind bekannt oder können nach bekannten Methoden hergestellt werden [EP-A 88 276; DE-A 28 47 236].

Die erfindungsgemäßen Verbindungen der allgemeinen Formel (Ia,b) sind Wirkstoffe für Arzneimittel. Insbesondere sind sie Inhibitoren der 3-Hydroxy-3-methyl-glutarylCoenzym A (HGM-CoA) Reduktase und infolgedessen Inhibitoren der Cholesterolbiosynthese. Sie können deshalb zur Behandlung von Hyperlipoproteinämie, Lipoproteinämie oder Arteriosklerose eingesetzt werden. Die erfindungsgemäßen Wirkstoffe bewirken außerdem eine Senkung des Cholesteringehaltes im Blut.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.b: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin- Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkam zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Im folgenden sind die erfindungsgemäßen Endprodukte mit (EP) und die erfindungsgemäßen Zwischenprodukte mit (ZP) gekennzeichnet.

### Herstellungsbeispiele

### Beispiel 1

### (E/Z)-4-Carboxyethyl-5-(4-fluorphenyl)-2-methyl-pent-4-en-3-on

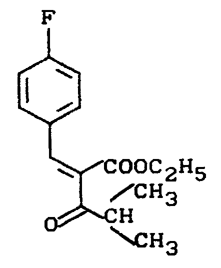

62 g (0,5 mol) 4-Fluorbenzaldehyd und 79 g (0,5 mol) Isobutanoylessigsäureethylester werden in 300 ml trockenem Isopropanol vorgelegt und mit einem Gemisch aus 2,81 ml (28 mmol) Piperidin und 1,66 ml (29 mmol) Essigsäure in 40 ml Isopropanol versetzt. Man läßt 48 h bei Raumtemperatur rühren, engt im Vakuum ein und destilliert den Rückstand im Hochvakuum.
- Kp 0,5 mm :: 127°C
- Ausbeute:: 108,7 g (82,3% der Theorie)

### Beispiel 2

### 1,4-Dihydro-2,6-diisopropyl-4-(4-fluorphenyl)-pyridin-3,5-dicarbonsäure-diethylester

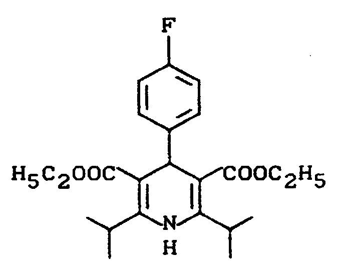

98 g (0,371 mol) der Verbindung aus Beispiel 1 werden mit 58,3 g (0,371 mol) 3-Amino-4-methyl-pent-2-en-säureethylester in 300 ml Ethanol 18 Stunden unter Rückfluß gekocht. Die Mischung wird auf Raumtemperatur abgekühlt, das Lösungsmittel im Vakuum abgedampft und die nicht umgesetzten Ausgangsmaterialien werden im Hochvakuum bei 130°C abdestilliert. Den zurückbleibenden Sirup verrührt man mit n-Hexan und saugt den ausgefallenen Niederschlag ab, wäscht mit n-Hexan nach und trocknet im Exsikkator.
- Ausbeute:: 35 g (23,4% der Theorie)
- ¹H-NMR (CDCl₃):: δ = 1,1 - 1,3 (m, 18H); 4,05 - 4,25 (m, 6H); 5,0 (s, (1H); 6,13 (s, 1H); 6,88 (m, 2H); 7,2 (m, 2H).

### Beispiel 3

### 2,6-Diisopropyl-4-(4-fluorphenyl)-pyridin-3,5-dicarbonsäure-diethylester

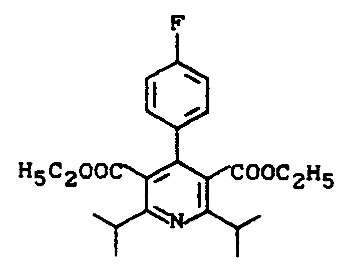

Zu einer Lösung von 6,6 g (16,4 mmol) der Verbindung aus Beispiel 2 in 200 ml Methylenchlorid p.a. gibt man 3,8 g (16,4 mmol) 2,3-Dichlor-5,6-dicyan-p-benzochinon und rührt 1 h bei Raumtemperatur. Dann wird über Kieselgur abgesaugt, die Methylenchloridphase dreimal mit je 100 ml Wasser extrahiert und über Magnesiumsulfat getrocknet. Nach Einengen im Vakuum wird der Rückstand an einer Säule (100 g Kieselgel 70-230 mesh, φ 3,5 cm, mit Essigester/Petrolether (1:9), chromatographiert.
- Ausbeute:: 5,8 g (87,9% der Theorie)
- ¹H-NMR (CDCl₃):: δ = 0,98 (t, 6H); 1,41 (d, 12H); 3,1 (m, 2H); 4,11 (q, 4H); 7,04 (m, 2H); 7,25 (m, 2H).

### Beispiel 4

### 3,5-Dihydroxymethyl-2,6-diisopropyl-4-(4-fluorphenyl)-pyridin

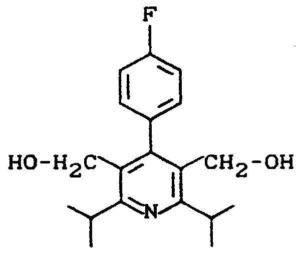

Unter Stickstoff gibt man zu einer Lösung von 4,6 g (11,4 mmol) der Verbindung aus Beispiel 3 in 100 ml trockenem Tetrahydrofuran bei -10°C bis -5°C 22,8 ml (80 mmol) einer 3,5 molaren Lösung von Natrium-bis-(2-methoxyethoxy)-dihydroaluminat in Toluol. Man rührt über Nacht bei Raumtemperatur und erwärmt anschließend 5 h auf 40°C. Nach erneutem Abkühlen auf 0°C tropft man vorsichtig 100 ml Wasser zu und extrahiert dreimal mit je 100 ml Essigester. Die vereinigten organischen Phasen werden mit gesättigter Natriumchlorid-Lösung gewaschen, über Magnesiumsulfat getrocknet und im Vakuum eingeengt. Der Rückstand wird an einer Säule (100 g Kieselgel 70-230 mesh, φ 3,5 cm, mit Essigester/Petrolether (6:4), chromatographiert.
- Ausbeute:: 2,4 g (66,7% der Theorie)
- ¹H-NMR (CDCl₃):: δ = 1,35 (d, 12H); 3,43 (m, 2H); 4,47 (d, 4H); 7,05 - 7,3 (m, 4H).

### Beispiel 5

### 2,6-Diisopropyl-4-(4-fluorphenyl)-pyridin-3,5-dicarbaldehyd

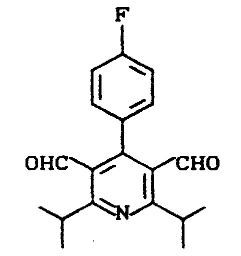

Zu einer Lösung von 3,8 g (12 mmol) der Verbindung aus Beispiel 4 in 100 ml Methylenchlorid p.a. gibt man 10,3 g (48 mmol) Pyridiniumchlorochromat und 4,9 g (48 mmol) neutrales Aluminiumoxid und rührt 1 h bei Raumtemperatur. Man saugt über Kieselgur ab und wäscht mit 300 ml Methylenchlorid nach. Die Methylenchloridphase wird im Vakuum eingeengt und der Rückstand an einer Säule (150 g Kieselgel 70-230 mesh, φ 3,5 cm, mit Essigester/Petrolether 2:8) chromatographiert.
- Ausbeute:: 3,2 g (85,3% der Theorie)
- ¹H-NMR (CDCl₃):: δ = 1,33 (d, 12H); 3,85 (m, 2H); 7,1 - 7,32 (m, 4H); 9,8 (s, 2H).

### Beispiel 6

### (E,E)-2,6-Diisopropyl-4-(4-fluorphenyl)-3,5-di(prop-2-en-1-al-3-yl)-pyridin

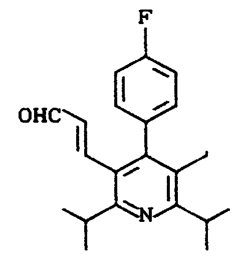

Unter Stickstoff tropft man zu einer Suspension von 0,36 g (12 mmol) 80%igem Natriumhydrid in 20 ml trockenem Tetrahydrofuran bei -5°C 3,1 g (12 mmol) Diethyl-2-(cyclohexylamino)-vinylphosphonat gelöst in 20 ml trockenem Tetrahydrofuran. Nach 30 min. werden bei derselben Temperatur 1,6 g (5 mmol) der Verbindung aus Beispiel 5 in 20 ml trockenem Tetrahydrofuran zugetropft und 30 min zum Rückfluß erwärmt. Nach Abkühlen auf Raumtemperatur wird der Ansatz in 200 ml eiskaltes Wasser gegeben und dreimal mit je 100 ml Essigester extrahiert. Die vereinigten organischen Phasen werden mit gesättigter Natriumchlorid-Lösung gewaschen und über Magnesiumsulfat getrocknet. Nach Einengen im Vakuum wird der Rückstand in 50 ml Toluol aufgenommen, mit einer Lösung von 2 g (15 mmol) Oxalsäure-Dihydrat in 25 ml Wasser versetzt und 30 min zum Rückfluß erwärmt. Nach Abkühlen auf Raumtemperatur werden die Phasen getrennt, die organische Phase mit gesättigter Natriumchlorid-Lösung gewaschen, über Magnesiumsulfat getrocknet und im Vakuum eingeengt. Der Rückstand wird an einer Säule (100 g Kieselgel 70-230 mesh, φ 3,5 cm, mit Essigester/Petrolether 2:8) chromatographiert.
- Ausbeute:: 920 mg (50% der Theorie)
- ¹H-NMR (CDCl₃):: δ = 1,33 (d, 12H); 3,33 (m, 2H); 6,03 (dd, 2H); 7,0 - 7,35 (m, 6H); 9,42 (d, 2H).

### Beispiel 7 (ZP)

### 2,6-Diisopropyl-4-(4-fluorphenyl)-3,5-di-(methyl-(E)-5-hydroxy-3-oxo-hept-6-enoat-7-yl)-pyridin

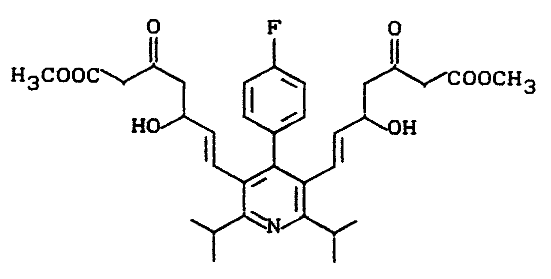

Unter Stickstoff tropft man zu einer Suspension von 360 mg (12 mmol) 80%igem Natriumhydrid in 30 ml trockenem Tetrahydrofuran bei -5°C 1,16 g (10 mmol) Acetessigsäuremethylester in 5 ml trockenem Tetrahyrofuran. Nach 15 min werden bei derselben Temperatur 6,2 ml (10 mmol) 15%iges Butyllithium in n-Hexan zugetropft und 15 min. nachgerührt. Anschließend werden 912 mg (2,5 mmol) der Verbindung aus Beispiel 6 gelöst in 20 ml trockenem Tetrahydrofuran zugetropft und 30 min bei -5°C nachgerührt. Die Reaktionslösung wird vorsichtig mit 3 ml 50%iger Essigsäure versetzt, mit 100 ml Wasser verdünnt und die Mischung dreimal mit je 100 ml Ether extrahiert. Die vereinigten organischen Phasen werden zweimal mit gesättigter Natriumhydrogencarbonat-Lösung und einmal mit gesättigter Natriumchlorid-Lösung gewaschen, über Magnesiumsulfat getrocknet und im Vakuum eingeengt. Der Rückstand wird an einer Säule (80 g Kieselgel 70-230 mesh, φ 3 cm, mit Essigester/Petrolether 1:1) chromatographiert.
- Ausbeute:: 800 mg (53,6% der Theorie)
- ¹H-NMR (CDCl₃):: δ = 1,25 (m, 12H); 2,47 (m, 4H); 3,25 (m, 2H); 3,42 (s, 4H); 3,73 (s, 6H); 4,5 (m, 2H); 5,25 (dd, 2H); 6,35 (dd, 2H); 7,0 (m, 4H).

### Beispiel 8 (EP)

### 2,6-Diisopropyl-4-(4-fluorphenyl)-3,5-di-(methyl-erythro-(E)-3,5-dihydroxy-hept-6-enoat-7-yl)-pyridin

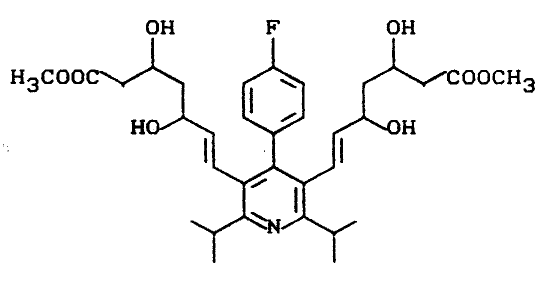

Zu einer Lösung von 776 mg (1,3 mmol) der Verbindung aus Beispiel 7 in 30 ml trockenem Tetrahydrofuran gibt man bei Raumtemperatur 3,2 ml (3,2 mmol) 1 M Triethylboran-Lösung in Tetrahydrofuran, leitet während 5 min Luft durch die Lösung und kühlt auf -30°C Innentemperatur ab. Es werden 122 mg (3,2 mmol) Natriumborhydrid und langsam 2,5 ml Methanol zugegeben, 30 min bei -30°C gerührt und dann mit einem Gemisch von 10 ml 30%igem Wasserstoffperoxid und 20 ml Wasser versetzt. Die Temperatur läßt man dabei bis 0°C ansteigen und rührt noch 30 min nach. Die Mischung wird dreimal mit je 50 ml Essigester extrahiert, die vereinigten organischen Phasen je einmal mit gesättigter Natriumhydrogencarbonat-Lösung und gesättigter Natriumchlorid-Lösung gewaschen, über Magnesiumsulfat getrocknet und im Vakuum eingeengt. Der Rückstand wird an einer Säule (50 g Kieselgel 230-400 mesh, φ 2,5 cm, mit Essigester/Petrolether 1:1) chromatograhiert.
- Ausbeute:: 400 mg (51,4% der Theorie)
- ¹H-NMR (CDCl₃):: δ = 12,5 (m, 12H); 1,4 (m, 4H); 2,42 (m, 4H); 3,3 (m, 2H); 3,72 (s, 6H); 4,1 (m, 2H); 4,3 (m, 2H); 5,25 (dd, 2H); 6,3 (dd, 2H); 7,0 (m, 4H).

### Beispiel 9

### (E/Z)-4-Carboxyethyl-2-methyl-5-phenyl-pent-4-en-3-on

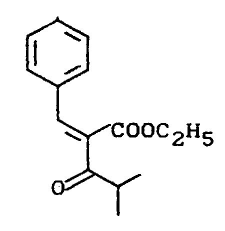

6,9 g (0,44 mol) Isobutanoylessigsäureethylester und 46,3 g (0,44 mol) Benzaldehyd werden in 300 ml Isopropanol vorgelegt, mit einem Gemisch aus 2,5 ml (25 mmol) Piperidin und 1,5 ml (26 mmol) Essigsäure in 40 ml Isopropanol versetzt und 24 h bei Raumtemperatur gerührt. Die Mischung wird im Vakuum eingeengt und der Rückstand im Hochvakuum destilliert.
- Kp 0,5 mm :: 130°C
- Ausbeute:: 60.7 g (56,2% der Theorie)

### Beispiel 10

### 1,4-Dihydro-2,6-diisopropyl-4-phenyl-pyridin-3,5-dicarbonsäure-diethylester

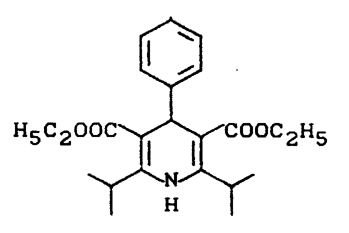

29,5 g (120 mmol) der Verbindung aus Beispiel 9 und 18,8 g (120 mmol) 3-Amino-4-methyl-pent-2-en-säureethylester werden in 150 ml Ethanol 48 Stunden unter Rückfluß gekocht. Die Mischung wird abgekühlt, im Vakuum eingeengt und der Rückstand an einer Säule (500 g Kieselgel, 70-230 mesh, φ 5 cm, mit Essigester/Petrolether 1:9) chromatographiert.
- Ausbeute:: 7,2 g (15,1% der Theorie)
- ¹H-NMR (CDCl₃):: δ = 1,2 (m, 18 H); 4,1 (m, 4H); 4,21 (m, 2H); 5,02 (s, 1H); 6,13 (s, 1H); 7,2 (m, 5H).

### Beispiel 11

### 2,6-Diisopropyl-4-phenyl-pyridin-3,5-dicarbonsäure-diethylester

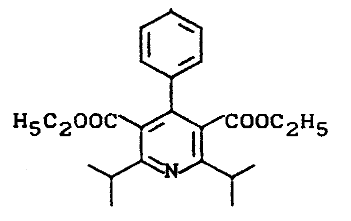

7,2 g (18,2 mmol) der Verbindung aus Beispiel 10 werden analog Beispiel 3 umgesetzt.
- Ausbeute:: 6,4 g (88,8% der Theorie)

### Beispiel 12

### 3,5-Dihydroxymethyl-2,6-diisopropyl-4-phenyl-pyridin

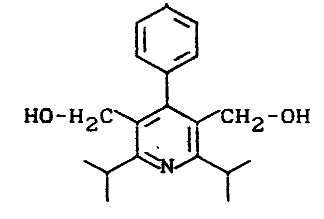

Unter Stickstoff werden zu einer Lösung von 6,4 g (16,2 mmol) der Verbindung aus Beispiel 11 in 100 ml trockenem Tetrahydrofuran bei 0°C 40,5 ml (40,5 mmol) einer 1 molaren Lösung von Lithiumaluminiumhydrid in Ether zugetropft. Man rührt über Nacht bei Raumtemperatur, erwärmt 3 h auf 50°C und kühlt erneut auf 0°C ab. Zu der Mischung tropft man vorsichtig 200 ml Wasser, saugt über Kieselgur ab und wäscht mit 250 ml Ether nach. Die organische Phase wird abgetrennt, einmal mit gesättigter Natriumchlorid-Lösung gewaschen, über Magnesiumsulfat getrocknet und im Vakuum eingeengt. Den Rückstand verrührt man mit Petrolether, saugt den Niederschlag ab und trocknet im Exsikkator.
- Ausbeute:: 4 g (83,3% der Theorie)
- ¹H-NMR (CDCl₃):: δ = 1,3 (d, 12H); 3,5 (m, 2H); 4,3 (d, 4H); 7,35 (m, 5H).

### Beispiel 13

### 2,6-Diisopropyl-4-phenyl-pyridin-3,5-dicarbaldehyd

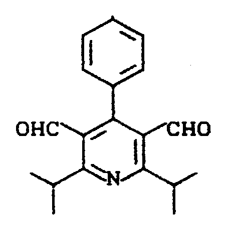

4 g (13,3 mmol) der Verbindung aus Beispiel 12 werden analog Beispiel 5 umgesetzt.
- Ausbeute:: 3,4 g (87,2% der Theorie)
- ¹H-NMR (CDCl₃):: δ = 1,35 (d, 12H); 3,4 (m, 2H); 7,3 (m, 2H); 7,5 (m, 3H); 9,3 (s, 2H).

### Beispiel 14

### (E,E)-2,6-Diisopropyl-4-phenyl-3,5-di-(prop-2-en-1-al-3-yl)-pyridin

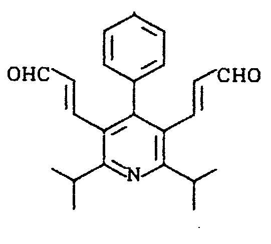

3,35 g (11,4 mmol) der Verbindung aus Beispiel 13 werden analog Beispiel 6 umgesetzt.
- Ausbeute:: 2,7 g (67,5% der Theorie)
- ¹H-NMR (CDCl₃):: δ = 1,35 (d, 12H); 3,47 (m, 2H); 6,04 (dd, 2H); 7,0 - 7,45 (m, 7H); 9,4 (d, 2H).

### Beispiel 15 (ZP)

### 2,6-Diisopropyl-4-pnenyl-3,5-di-(methyl-(E)-5-hydroxy-3-oxo-hept-6-enoat-7-yl)-pyridin

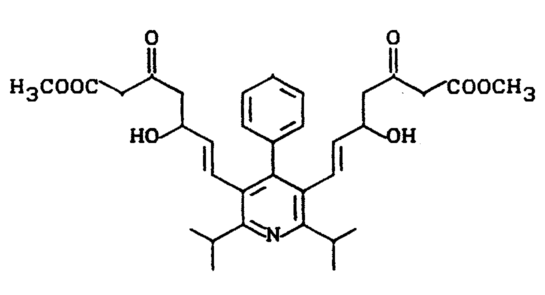

2,7 g (7,8 mmol) der Verbindung aus Beispiel 14 werden analog Beispiel 7 umgesetzt.
- Ausbeute:: 3,2 g (71,1% der Theorie)
- ¹H-NMR (CDCl₃):: δ = 1,25 (m, 12H); 2,35 (m, 4H); 3,27 (m, 2H); 3,40 (s, 4H); 3,75 (s, 6H); 4,38 (m, 2H); 5,25 (dd, 2H); 6,37 (dd, 2H); 7,02 (m, 2H); 7,30 (m, 3H).

### Beispiel 16 (EP)

### 2,6-Diisopropyl-4-phenyl-3,5-di-(methyl-erythro-(E)-3,5-dihydroxy-hept-6-enoat-7-yl)-pyridin

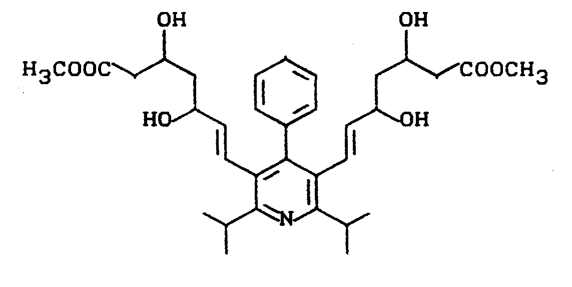

3,2 g (5,5 mmol) der Verbindung aus Beispiel 15 werden analog Beispiel 8 umgesetzt.
- Ausbeute:: 2 g (62,5% der Theorie)
- ¹H-NMR (CDCl₃):: δ = 1,25 (m, 12H); 1,3 - 1,7 (m, 4H); 2,39 (m, 4H); 3,2 - 3,4 (m, 2H); 3,71 (s, 6H); 4,02 (m, 2H); 4,27 (m, 2H); 5,3 (m, 2H); 6,32 (m, 2H); 7,0 (m, 2h); 7,25 (m, 3H).

### Beispiel 17

### (E/Z)-2-Ethoxycarbonyl-1-(4-fluorphenyl)-but-2-en-3-on

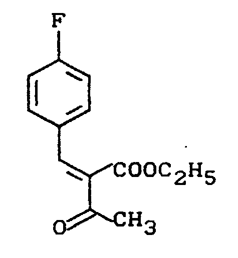

62 g (0,5 mol) 4-Fluorbenzaldehyd und 53,9 ml (0,5 mol) Acetessigsäuremethylester werden in 300 ml Isopropanol vorgelegt, mit einem Gemisch aus 2,81 ml (28 mmol) Piperidin und 1,66 ml (29 mmol) Essigsäure in 40 ml Isopropanol versetzt und 48 h bei Raumtemperatur gerührt. Die Mischung wird im Vakuum eingeengt und der Rückstand im Hochvakuum destilliert.
- Kp 0,5 mm:: 138°C
- Ausbeute:: 50,5 g (45,5% der Theorie)

### Beispiel 18

### 1,4-Dihydro-2,6-dimethyl-4-(4-fluorphenyl)-pyridin-3,5-dicarbansäure-dimethylester

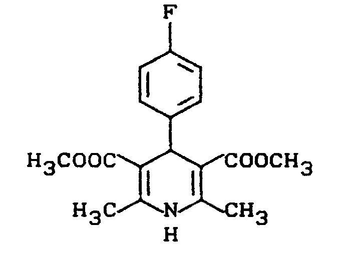

33,3 g (0,15 mol) der Verbindung aus Beispiel 17 werden mit 17,3 g (0,15 mol) 3-Aminocratansäuremethylester in 150 ml Ethanol 4 h unter Rückfluß gekocht. Die Mischung wird auf 0°C abgekühlt, der ausgefallene Niederschlag abgesaugt, mit wenig Petrolether nachgewaschen und im Exsikkator getrocknet.
- Ausbeute:: 32 g (66,8% der Theorie)
- ¹H-NMR (CDCl₃):: δ = 2,33 (s, 6H); 3,65 (s, 6H); 4,99 (s, 1H); 5,77 (s, 1H) 6,89 (m, 2H); 7,22 (m, 2H).

### Beispiel 19

### 2,6-Dimethyl-4-(4-fluorphenyl)-pyridin-3,5-dicarbonsäure-dimethylester

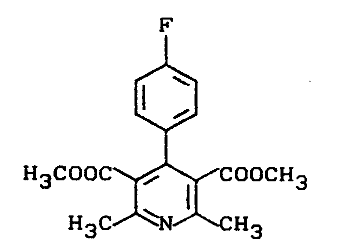

32 g (0,1 mol) der Verbindung aus Beispiel 18 werden analog Beispiel 3 umgesetzt.
- Ausbeute:: 27,2 g (87% der Theorie)
- ¹H-NMR (CDCl₃):: δ = 2,59 (s, 6H); 3,56 (s, 6H); 7,08 (m, 2H); 7,25 (m, 2H).

### Beispiel 20

### 3,5-Dihydroxymethyl-2,6-dimethyl-4-(4-fluorphenyl)-pyridin

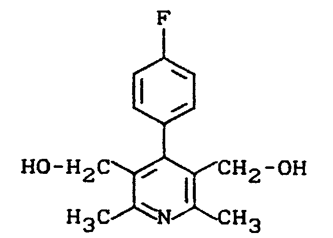

Unter Stickstoff gibt man zu einer Lösung von 7,9 g (25 mmol) der Verbindung aus Beispiel 19 in 100 ml trockenem Tetrahydrofuran bei 0°C 25 ml (87,5 mmol) einer 3,5 molaren Lösung von Natrium-bis-(2-methoxyethoxy)-dihydroaluminat in Toluol. Man rührt 6 h bei Raumtemperatur, kühlt erneut auf 0°C ab und tropft langsam 200 ml Wasser zu. Die Mischung wird dreimal mit je 150 ml Essigester extrahiert, die vereinigten organischen Phasen einmal mit gesättigter Natriumchlorid-Lösung gewaschen, über Magnesiumsulfat getrocknet und im Vakuum eingeengt. Den Rückstand verrührt man mit Ether, saugt ab und trocknet im Exsikkator.
- Ausbeute:: 3,5 g (53,8% der Theorie)
- ¹H-NMR (CDCl₃):: δ = 2,81 (s, 6H); 4,28 (d, 4H); 7,1 (m, 2H); 7,3 (m, 2H).

### Beispiel 21

### 2,6-Dimethyl-4-(4-fluorphenyl)-pyridin-3,5-carbaldehyd

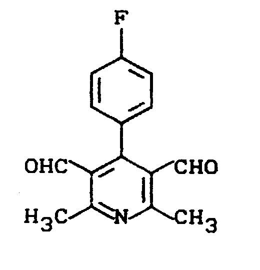

3,5 g (13,4 mmol) der Verbindung aus Beispiel 20 werden analog Beispiel 5 umgesetzt.
- Ausbeute:: 1,7 g (53% der Theorie)
- ¹H-NMR (CDCl₃):: δ = 2,88 (s, 6H); 7,28 (m, 4H); 9,82 (s, 2H).

### Beispiel 22

### (E,E)-2,6-Dimethyl-4-(4-fluorphenyl)-3,5-bis-(prop-en-1-al-3-yl)-pyridin

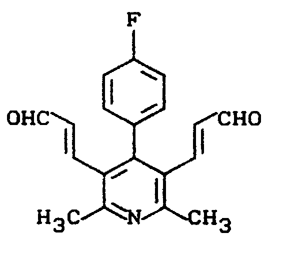

1,7 g (7 mmol) der Verbindung aus Beispiel 21 werden analog Beispiel 6 umgesetzt.
- Ausbeute:: 1 g (47,6% der Theorie)
- ¹H-NMR (CDCl₃):: δ = 2,7 (s, 6H); 6,15 (dd, 2H); 7,15 (m, 6H); 9,43 (d, 2H).

### Beispiel 23

### 2,6-Dimethyl-4-(4-fluorphenyl)-3,5-di-(methyl-(E)-5-hydroxy-3-oxo-hept-6-enoat-7-yl)-pyridin

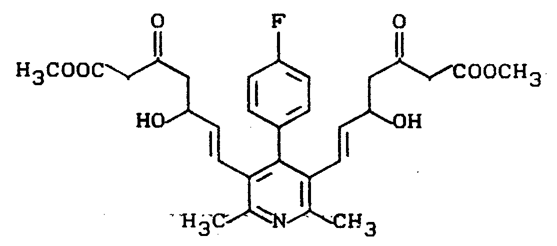

1 g (3,2 mmol) der Verbindung aus Beispiel 22 werden analog Beispiel 7 umgesetzt.
- Ausbeute:: 1 g (57 % der Theorie)
- ¹H-NMR (CDCl₃):: δ = 2,50 (m, 4H); 2,55 (s, 6H); 3,45 (s, 4H); 3,73 (s, 6H); 4,52 (m, 2H); 5,35 (dd, 2H); 6,28 (d, 2H); 7,05 (m, 4H) ppm.

### Beispiel 24 (EP)

### 2,6-Dimethyl-4-(4-fluorphenyl)-3,5-di-(methyl-erythro-(E)-3,5-dihydroxy-hept-6-enoat-7-yl)-pyridin

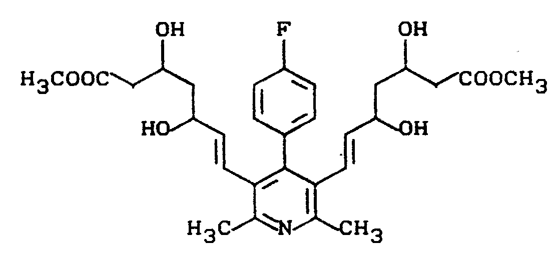

1 g (1,8 mmol) der Verbindung aus Beispiel 23 werden analog Beispiel 8 umgesetzt.
- Ausbeute:: 0,7 g (69,9 % der Theorie)
- ¹H-NMR (CDCl₃):: δ = 1,20-1,60 (m, 4H); 2,43 (m, 4H); 2,54 (s, 6H); 3,72 (s, 6H); 4,09 (m, 2H); 4,33 (m, 2H); 5,37 (dd, 2H); 6,22 (d, 2H); 7,03 (m, 4H) ppm.

### Beisiel 25

### 1,4-Dihydro-4-(4-fluorphenyl)-2-isopropyl-6-methyl-pyridin-3,5-dicarbonsäure-3-ethyl-5-methylester

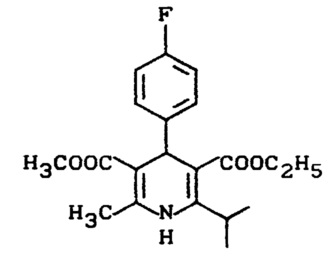

15 g (56,8 mol) der Verbindung aus Beispiel 1 und 6,5 g (56,8 mmol) 3-Aminocrotonsäuremethylester werden in 150 ml Ethanol 20 h am Rückfluß gekocht. Die Mischung wird abgekühlt, abfiltriert und im Vakuum eingeengt. Der Rückstand wird an einer Säule (250 g Kieselgel 70-230 mesh, φ 4,5 cm, mit Essigester/Petrolether 3:7) chromatographiert.
- Ausbeute:: 13,6 g (66,3% der Theorie)
- ¹H-NMR (CDCl₃):: δ = 1,2 (m, 9H); 2,35 (s, 3H); 3,65 (s, 3H); 4,12 (m, 3H); 4,98 (s, 1H); 5,75 (s, 1H); 6,88 (m, 2H); 7,25 (m, 2H).

### Beispiel 26

### 4-(4-Fluorphenyl)-2-isopropyl-6-methyl-pyridin-3,5-dicarbonsäure-3-ethyl-5-methylester

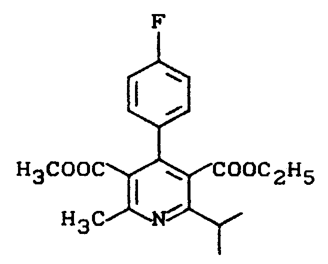

13,5 g (37,4 mmol) der Verbindung aus Beispiel 25 werden analog Beispiel 3 umgesetzt.
- Ausbeute:: 9,5 g (70,9% der Theorie)
- ¹H-NMR (CDCl₃):: δ = 0,98 (t, 3H); 1,31 (d, 6H); 2,6 (s, 3H); 3,11 (m, 1H); 3,56 (s, 3H); 4,03 (q, 2H); 7,07 (m, 2H); 7,25 (m, 2H).

### Beispiel 27

### 3,5-Dihydroxymethyl-4-(4-fluorphenyl)-2-isopropyl-6-methyl-pyridin

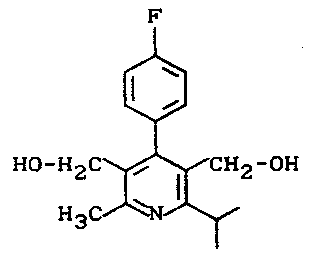

Unter Stickstoff werden zu einer Suspension von 6 g (158 mmol) Lithiumaluminiumhydrid in 200 ml absolutem Tetrahydrofuran bei 60°C langsam 20,7 g (57,7 mmol) der Verbindung aus Beispiel 26 gelöst in 50 ml absolutem Tetrahydrofuran zugetropft. Die Mischung wird 1 h zum Rückfluß erhitzt, auf 0°C abgekühlt und vorsichtig mit 18 ml Wasser versetzt. Man gibt 6 ml 10%ige Kaliumhydroxyd-Lösung dazu, saugt vom Niederschlag ab und kocht den Rückstand zweimal mit je 250 ml Ether aus. Die vereinigten Mutterlaugen werden über Magnesiumsulfat getrocknet und im Vakuum eingeengt. Der Rückstand wird mit Petrolether verrührt, abgesaugt und im Exsikkator getrocknet.
- Ausbeute:: 11,5g (68,9% der Theorie)
- ¹H-NMR (CDCl₃):: δ = 1,3 (d, 6H); 2,69 (s, 3H); 3,51 (m, 1H); 4,3 (m, 4H); 7,1 (m, 2H); 7,3 (m, 2H).

### Beispiel 28

### 4-(4-Fluorphenyl)-2-isopropyl-6-methyl-pyridin-3,5-dicarbaldehyd

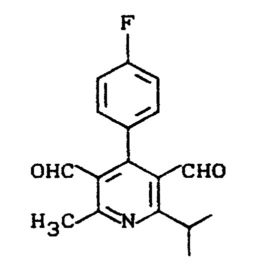

11,5 g (40 mmol) der Verbindung aus Beispiel 27 werden analog Beispiel 5 umgesetzt.
- Ausbeute:: 7,3 g (64% der Theorie)
- ¹H-NMR (CDCl₃):: δ = 1,32 (d, 6H); 2,88 (s, 3H); 3,87 (m, 1H); 7,25 (m, 4H); 9,81 (d, 2H).

### Beispiel 29

### (E,E)-4-(4-Fluorphenyl)-2-isopropyl-6-methyl-3,5-di-(prop-2-en-1-al-3-yl)-pyridin

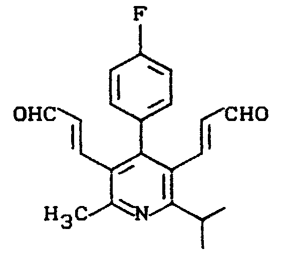

7,3 g (21,7 mmol) der Verbindung aus Beispiel 28 werden analog Beispiel 6 umgesetzt.
- Ausbeute:: 6,35 g (86,9% der Theorie)
- ¹H-NMR (CDCl₃):: δ = 1,32 (d, 6H); 2,7 (s, 3H); 3,34 (m, 1H); 6,02 (dd, 1H); 6,15 (dd, 1H); 7,0 - 7,35 (m, 6H); 9,42 (d, 1H); 9,43 (d, 1H).

### Beispiel 30 (ZP)

### 4-(4-Fluorphenyl)-2-isopropyl-6-methyl-3,5-di-(methyl-(E)-5-hydroxy-3-oxo-hept-6-enoat-7-yl)-pyridin

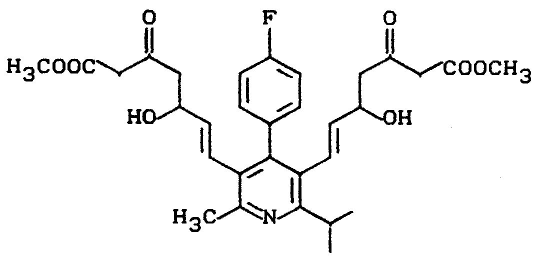

6,3 g (18,8 mol) der Verbindung aus Beispiel 29 werden analog Beispiel 7 umgesetzt.
- Ausbeute:: 5,7 g (53,5% der Theorie)
- ¹H-NMR (CDCl₃):: δ = 1,25 (d, 6H); 2,42 (m, 2H); 2,53 (m, 2H); 2,57 (s, 3H); 3,28 (m, 1H); 3,42 (s, 2H); 3,43 (s, 2H); 3,75 (s, 6H); 4,52 (m, 2H); 5,25 (dd, 1H); 5,36 (dd, 1H); 6,28 (d, 1H); 6,37 (d, 1H); 6,90 - 7,20 (m, 4H).

### Beispiel 31 (EP)

### 4-(4-Fluorphenyl)-2-isopropyl-6-methyl-3,5-di-(methyl-erythro-(E)-3,5-dihydroxy-hept-6-enoat-7-yl)-pyridin

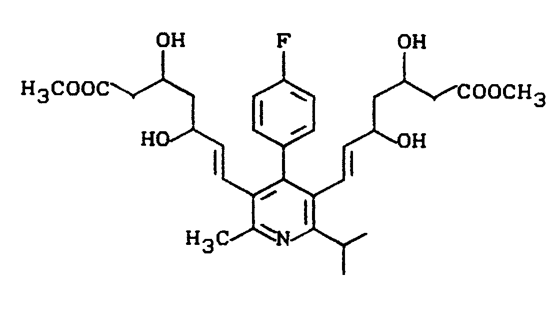

5,7 g (10 mmol) der Verbindung aus Beispiel 30 werden analog Beispiel 8 umgesetzt.
- Ausbeute:: 2,9 g (50,8% der Theorie)
- ¹H-NMR (CDCl₃):: δ = 1,25 (m, 6H); 1,2 - 1,5 (m, 4H); 2,41 (m, 4H); 2,57 (s, 3H); 3,3 (m, 1H); 3,72 (s, 6H); 4,1 (m, 2H); 4,3 (m, 2H); 5,15 - 5,5 (m, 2H); 6,15 - 6,45 (m, 2H); 7,0 (m, 4H).

### Beispiel 32

### 1,4-Dihydro-2-ethyl-4-(4-fluorphenyl)-6-isopropyl-pyridin-3,5-dicarbonsäure-diethylester

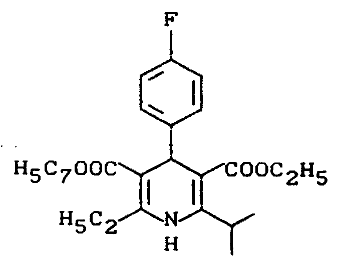

9.5 g (73,6 mmol) 3-Amino-pent-2-en-säuremethylester und 19,4 g (73,6 mmol) der Verbindung aus Beispiel 1 werden in 200 ml n-Butanol 5 Stunden unter Rückfluß gekocht. Die Mischung wird auf Raumtemperatur abgekühlt, das Lösungsmittel im Vakuum eingeengt und der Rückstand an einer Säule (Kieselgel 70-230 mesh, mit Petrolether/Essigester 9:1) chromatographiert.
- Ausbeute:: 5,3 g (19,3 % der Theorie)

### Beispiel 33 (EP)

### 2-Ethyl-4-(4-fluorphenyl)-6-isopropyl-3,5-di-(methyl-erythro-(E)-3,5-dihydroxy-hept-6-enoat-7-yl)-pyridin

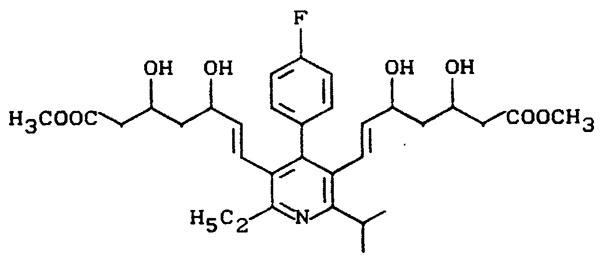

Aus der Verbindung aus Beispiel 32 wurde, in Analogie zu den Reaktionen aus den Beispielen 3, 27, 5, 6, 7, 8, das Beispiel 33 hergestellt:
- ¹H-NMR (CDCl₃):: δ = 1,28 (m, 9H); 1,43 (m, 4H); 2,42 (m, 4H); 2,82 (q, 2H); 3,32 (m, 1H); 3,72 (s, 6H); 4,09 (m, 2H); 4,32 (m, 2H); 5,28 (m, 2H); 6,30 (m, 2H); 7,00 (m, 4H) ppm

### Beispiel 34

### 1,4-Dihydro-4-(4-fluorphenyl)-2-isopropyl-6-n-propyl-pyridin-3,5-dicarbonsäure-diethylester

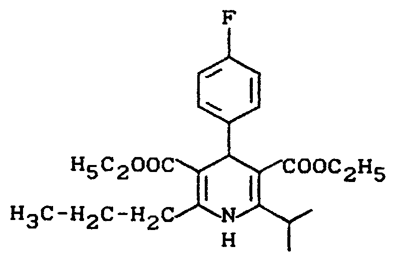

5,8 g (36,8 mmol) 3-Amino-hex-2-en-säureethylester und 9,7 g (36,8 mmol) der Verbindung aus Beispiel 1 werden in 100 ml Ethanol p.A. 48 Stunden unter Rückfluß gekocht. Die Mischung wird abgekühlt, das Lösungsmittel im Vakuum eingeengt und der Rückstand an einer Säule (Kieselgel 70-230 mesh, mit Petrolether/Essigester 8:2) chromatographiert.
- Ausbeute:: 2,6 g (17,7 % der Theorie).

### Beispiel 35 (EP)

### 4-(4-Fluorphenyl)-2-isopropyl-6-n-propyl-3,5-di-(methyl-erythro-(E)-3,5-dihydroxy-hept-6-enoat-7-yl)-pyridin

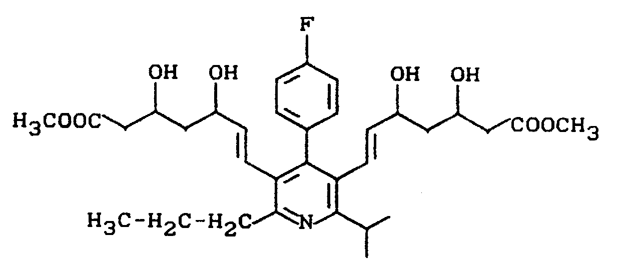

Aus der Verbindung aus Beispiel 34 wurde, in Analogie zu den Reaktionen aus den Beispielen 3, 27, 5, 6, 7, 8 das Beispiel 35 hergestellt.
- ¹H-NMR (CDCl₃):: δ = 0,98 (t, 3H); 1,22 (d, 6H); 1,38 (m, 4H); 1,77 (m, 2H); 2,40 (m, 4H); 2,78 (m, 2H); 3,28 (m, 1H); 3,70 (s, 6H); 4,05 (m, 2H); 4,28 (m, 2H); 5,25 (m, 2H); 6,28 (m, 2H); 6,95 (m, 4H) ppm.

### Beispiel 36

### 1,4-Dihydro-2-n-butyl-4-(4-fluorphenyl)-6-isopropyl-pyridin-3,5-dicarbonsäure-diethylester

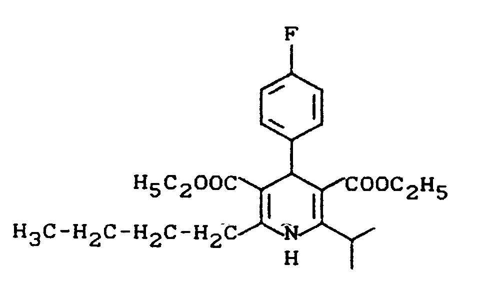

6,3 g (36,8 mmol) 3-Amino-hept-2-en-säureethylester und 9,7 g (36,8 mmol) der Verbindung aus Beispiel 1 werden analog Beispiel 34 umgesetzt.
- Ausbeute:: 2,5 g (16,4 % der Theorie)

### Beispiel 37 (EP)

### 2-n-Butyl-4-(4-fluorphenyl)-6-isopropyl-3,5-di-(methyl-erythro-(E)-3,5-dihydroxy-hept-6-enoat-7-yl)-pyridin

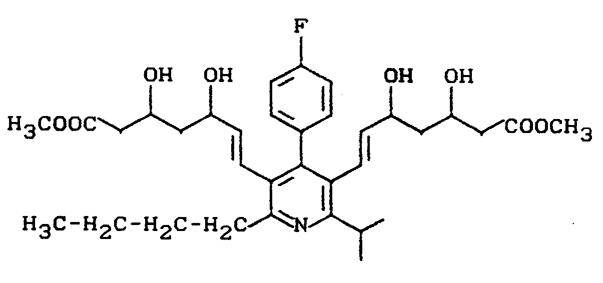

Aus der Verbindung aus Beispiel 36 wurde, in Analogie zu den Reaktionen aus den Beispielen 3, 27, 5, 6, 7, 8 das Beispiel 37 hergestellt.

### Beispiel 38

### 1,4-Dihydro-2-benzyl-4-(4-fluorphenyl)-6-isopropyl-pyridin-3,5-dicarbonsäure-diethylester

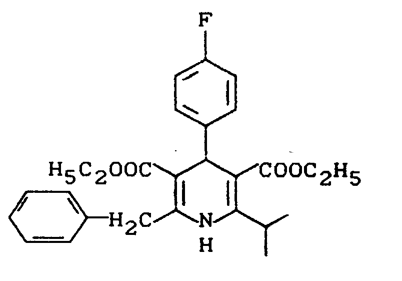

7,4 g (36,8 mmol) 3-Amino-4-phenyl-crotonsäureethylester und 9,7 g (36,8 mmol) der Verbindung aus Beispiel 1 werden analog Beispiel 34 umgesetzt.
- Ausbeute:: 2,1 g (12,6 % der Theorie).

### Beispiel 39 (EP)

### 2-Benzyl-4-(4-fluorphenyl)-6-isopropyl-3,5-di-(methyl-erythro-(E)-3,5-dihydroxy-hept-6-enoat-7-yl)-pyridin

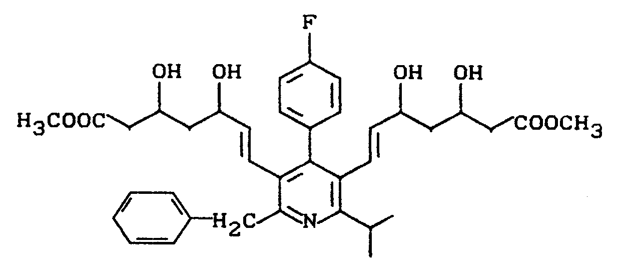

Aus der Verbindung aus Beispiel 38 wurde, in Analogie zu den Reaktionen aus den Beispielen 3, 27, 5, 6, 7, 8, das Beispiel 39 hergestellt.
- ¹H-NMR (CDCl₃):: δ = 1,10-1,50 (m, 10H); 2,40 (m, 4H); 3,32 (m, 1H); 3,72 (s, 3H); 3,73 (s, 3H); 3,98 (m, 1H); 4,08 (m, 1H); 4,18 (m, 1H); 4,21 (s, 2H); 4,28 (m, 1H); 5,13 (dd, 1H); 5,27 (dd, 1H); 6,25 (d, 1H); 6,33 (d, 1H); 6,97 (m, 4H); 7,25 (m, 5H) ppm.

### Beispiel 40

### (E/Z)-2-Carboxyethyl-1-cyclopropyl-3-(4-fluorphenyl)-prop-2-en-1-on

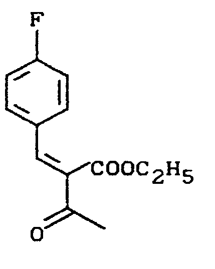

39 g (0,25 mol) Cyclopropylcarbonylessigsäureethylester und 31 g (0,25 mol) 4-Fluorbenzaldehyd werden in 150 ml trockenem Isopropanol vorgelegt und mit einem Gemisch aus 1,4 ml (14 mmol) Piperidin und 0,83 ml (14,5 mmol) Essigsäure in 20 ml Isopropanol versetzt. Man rührt 48 Stunden bei Raumtemperatur, engt im Vakuum ein und destilliert den Rückstand im Hochvakuum.
- Kp 0,5 mm:: 140°C
- Ausbeute:: 52,3 g (79,8 % der Theorie)

### Beispiel 41

### 1,4-Dihydro-2-cyclopropyl-4-(4-fluorphenyl)-6-isopropyl-pyridin-3,5-dicarbonsäure-diethylester

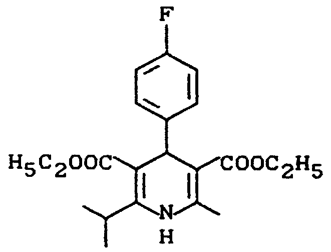

39,3 g (0,15 mol) der Verbindung aus Beispiel 40 und 23,6 g (0,15 mol) 3-Amino-4-methyl-pent-2-en-säureethylester werden in 150 ml Ethylenglykol über Nacht unter Rückfluß gekocht. Nach Abkühlen auf Raumtemperatur wird mehrmals mit Ether extrahiert, die vereinigten Etherphasen dreimal mit 10 %iger Salzsäure, je einmal mit Wasser und gesättigter Natriumhydrogencarbonat-Losung gewaschen, über Magnesiumsulfat getrocknet und im Vakuum eingeengt. Den Rückstand verrührt man mit Petrolether/Ether, saugt ab und trocknet im Exsikkator.
- Ausbeute:: 22,8 g (37,9 % der Theorie)
- ¹H-NMR (CDCl₃):: δ = 0,65 (m, 2H); 1,03 (m, 2H); 1,15 (m, 13H); 2,78 (m, 1H); 4,15 (m, 4H); 5,03 (s, 1H); 5,72 (s, 1H); 6,90 (m, 2H); 7,22 (m, 2H) ppm.

### Beispiel 42 (EP)

### 2-Cyclopropyl-4-(4-fluorphenyl)-6-isopropyl-3,5-di-(methyl-erythro-(E)-3,5-dihydroxy-hept-6-enoat-7-yl)-pyridin

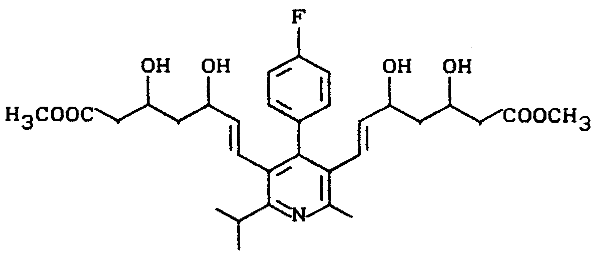

Aus der Verbindung aus Beispiel 41 wurde, in Analogie zu den Reaktionen aus den Beispielen 3, 27, 5, 6, 7, 8 das Beispiel 42 hergestellt.
- ¹H-NMR (CDCl₃):: δ = 0,90 (m, 2H); 1,20 (d, 6H); 1,10-1,60 (m, 6H); 2,25 (m, 1H); 2,43 (m, 4H); 3,25 (m, 1H); 3,73 (s, 6H); 4,08 (m, 2H); 4,30 (m, 2H); 5,22 (dd, 1H); 5,53 (dd, 1H); 6,30 (m, 2H); 6,98 (m, 4H) ppm.

### Beispiel 43

### 3-Amino-3-cyclopropyl-acrylsäureethylester

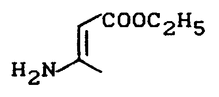

49,9 g (0,32 mol) Cyclopropylcarbonylessigsäureethylester in 200 ml trockenem Toluol werden mit 1,1 g p-Toluolsulfonsäure versetzt und bei Raumtemperatur unter Rühren mit Ammoniak-Gas gesättigt. Nach Stehenlassen über Nacht wird 8 Stunden am Wasserabscheider unter Rückfluß gekocht, wobei kontinuierlich Ammoniak-Gas eingeleitet wird. Man läßt über Nacht abkühlen, filtriert, engt die Toluollösung im Vakuum ein und destilliert im Hochvakuum bis 65°C vom nicht umgesetzten Ausgangsmaterial ab. Die Substanz befindet sich anschließend im Rückstand.
- Ausbeute:: 11,9 g (24 % der Theorie).

### Beispiel 44

### 1,4-Dihydro-2,6-dicyclopropyl-4-(4-fluorphenyl)-pyridin-3,5-dicarbonsäure-diethylester

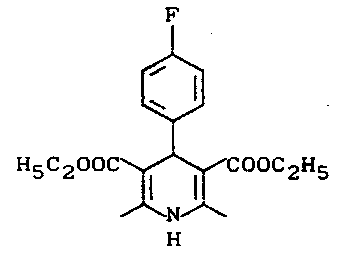

6,2 g (40 mmol) der Verbindung aus Beispiel 43 und 10,5 g (40 mmol) der Verbindung aus Beispiel 40 werden in 100 ml Ethylenglykol gelöst und über Nacht am Rückfluß gekocht. Nach Abkühlen auf Raumtemperatur wird die Mischung mehrmals mit Ether extrahiert, die organische Phase je einmal mit 10 %iger Salzsäure, gesättigter Natriumbicarbonat-Lösung und Wasser gewaschen, über Magnesiumsulfat getrocknet und im Vakuum eingeengt.
- Ausbeute:: 10,4 g (65,1 % der Theorie)
- ¹H-NMR (CDCl₃):: δ = 0,60 (m, 4H); 0,95 (m, 4H); 1,23 (t, 6H); 2,72 (m, 2H); 4,12 (m, 4H); 5,02 (s, 1H); 5,40 (s, 1H); 6,88 (m, 2H); 7,20 (m, 2H) ppm.

### Beispiel 45 (EP)

### 2,6-Dicyclopropyl-4-(4-fluorphenyl)-3,5-di-(methyl-erythro-(E)-3,5-dihydroxy-hept-6-enoat-7-yl)-pyridin

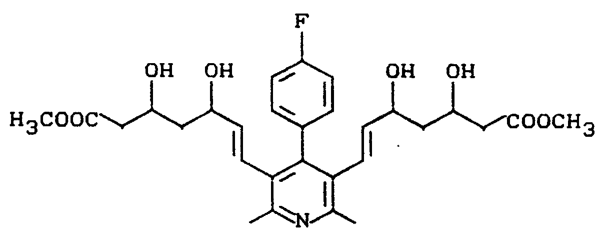

Aus der Verbindung aus Beispiel 44 wurde, in Analogie zu den Reaktionen aus den Beispielen 3, 27, 5, 6, 7, 8, das Beispiel 45 hergestellt.
- ¹H-NMR (CDCl₃):: δ = 0,85 (m, 4H); 1,08 (m, 4H); 1,20-1,60 (m, 4H): 2,20 (m, 2H); 2,43 (m, 4H); 3,70 (s, 6H); 4,12 (m, 2H); 4,33 (m, 2H); 5,52 (dd, 2H); 6,30 (d, 2H); 7,0 (m, 4H) ppm.

### Beispiel 46

### (E/Z)-4-Carboxyethyl-5-(4-fluor-3-phenoxyphenyl)-2-methyl-pent-4-en-3-on

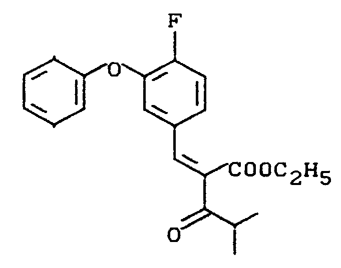

49 g (0,31 mol) Isobutanoylessigsäureethylester und 67 g (0,31 mol) 3-Phenoxy-4-fluorbenzaldehyd werden in 300 ml Isopropanol vorgelegt und mit einem Gemisch aus 1,81 ml (18 mmol) Piperidin und 1,06 ml (18,6 mmol) Essigsäure in 30 ml Isopropanol versetzt. Man rührt über Nacht bei Raumtemperatur, engt dann im Vakuum ein und trocknet im Hochvakuum.
- Ausbeute:: 110 g (wurde ohne weitere Reinigung in Beispiel 47 eingesetzt).

### Beispiel 47

### 1,4-Dihydro-2,6-diisopropyl-4-(4-fluor-3-phenoxyphenyl )pyridin-3,5-dicarbonsäure-diethylester

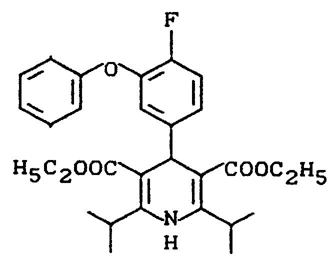

30 g (84,3 mmol) der Verbindung aus Beispiel 46 und 13,2 g (84,3 mmol) 3-Amino-4-methyl-pent-2-en-säureethylester werden in 150 ml Ethanol über Nacht am Rückfluß gekocht. Man kühlt die Mischung auf 0°C, saugt den ausgefallenen Niederschlag ab, wäscht mit Petrolether nach und trocknet im Exsikkator.
- Ausbeute:: 18,4 g (44,2 % der Theorie)
- ¹H-NMR (CDCl₃):: δ = 1,05-1,25 (m, 18H); 4,05-4,2 (m, 6H); 4,95 (s, 1H); 6,03 (s, 1H); 6,85-7,1 (m, 6H); 7,3 (m, 2H).

### Beispiel 48 (EP)

### 2,6-Diisopropyl-4-(4-fluor-3-phenoxyphenyl)-3,5-di-(methyl-erythro-(E)-3,5-dihydroxy-hept-6-enoat-7-yl)-pyridin

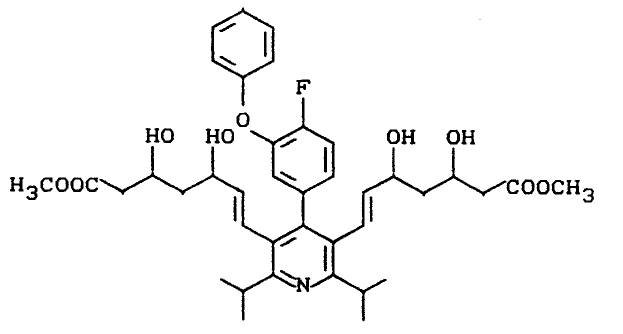

Aus der Verbindung aus Beispiel 47 wurde, in Analogie zu den Reaktionen aus den Beispielen 3, 27, 5, 6, 7, 8, das Beispiel 48 hergestellt.

### Beispiel 49

### (E/Z)-2-Carboxyethyl-1-(4-fluorphenyl)-3-phenyl-propen-3-on

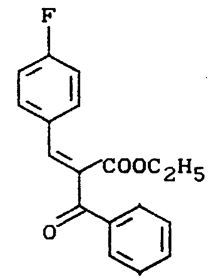

38,4 g (0,2 mol) Benzoylessigsäureethylester und 24,8 g (0,2 mol) 4-Fluorbenzaldehyd werden in 200 ml Toluol gelöst, mit 3 ml Piperidin und 3,5 ml Eisessig versetzt und über Nacht am Wasserabscheider unter Rückfluß erhitzt. Nach Abkühlen auf Raumtemperatur, extrahiert man mit gesättigter Natriumhydrogencarbonat-Lösung und Wasser, trocknet die organische Phase über Magnesiumsulfat und engt im Vakuum ein. Die nicht umgesetzten Ausgangsmaterialien werden im Hochvakuum abdestilliert und man erhält 55,9 g (93 % der Theorie) Rohprodukt im Destillationsrückstand.
- ¹H-NMR (CDCl₃):: δ = 1,15 (t, 3H); 4,21 (q, 2H); 6,85-7,95 (m, 10H) ppm.

### Beispiel 50

### 1,4-Dihydro-4-(4-fluorphenyl)-2-isopropyl-6-phenyl-pyridin-3,5-dicarbonsäure-diethylester

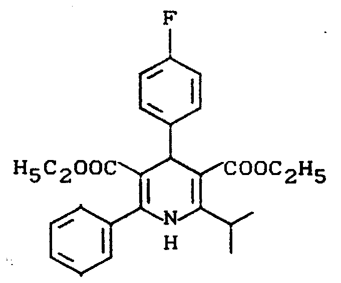

29,8 g (0,1 mol) der Verbindung aus Beispiel 49 und 15,7 g (0,1 mol) 3-Amino-4-methyl-pent-2-en-säureethylester werden in 150 ml Ethylenglykol gelöst und über Nacht am Rückfluß gekocht. Nach Einengen im Vakuum wird der Rückstand in Essigester gelöst, je einmal mit 10 %iger Salzsäure, gesättigter Natriumhydrogencarbonat-Lösung und Wasser gewaschen, über Magnesiumsulfat getrocknet und im Vakuum eingeengt. Der Rückstand wird an einer Säule (Kieselgel 70-230 mesh, mit Essigester/Petrolether 1:5) chromatographiert.
- Ausbeute:: 8,6 g (19,7 % der Theorie)
- ¹H-NMR (CDCl₃):: δ = 0,85 (t, 3H); 1,20 (m, 9H); 3,85 (q, 2H); 4,12 (q, 2H); 4,25 (m, 1H); 5,09 (m, 1H); 5,93 (m, 1H); 6,93 (m, 2H); 7,25-7,50 (m, 7H) ppm.

### Beispiel 51 (EP)

### 4-(4-Fluorphenyl)-2-isopropyl-6-phenyl-3,5-di-(methyl-erythro-(E)-3,5-dihydroxy-hept-6-enoat-7-yl)-pyridin

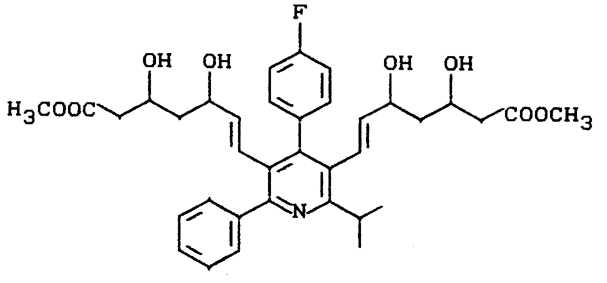

Aus der Verbindung aus Beispiel 50 wurde, in Analogie zu den Reaktionen aus den Beispielen 3, 27, 5, 6, 7, 8, das Beispiel 51 hergestellt.

### Beispiel 52

### (E/Z)-2-(Carboxy-2-cyanethyl)-3-cyclohexyl-1-(4-fluorphenyl)-propen-3-on

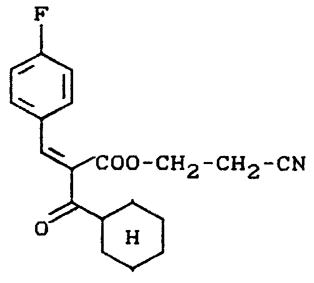

66,9 g (0,3 mol) Cyclohexylcarbonyl-essigsäure-(2-cyanethylester) und 37,2 g (0,3 mol) 4-Fluorbenzaldehyd wurden analog Beispiel 49 umgesetzt.
- Ausbeute:: 56,7 g (57,4 % der Theorie)

### Beispiel 53

### 1,4-Dihydro-2-cyclohexyl-4-(4-fluorphenyl)-6-isopropyl-pyridin-3,5-dicarbonsäure-3-cyanethylester-5-ethylester

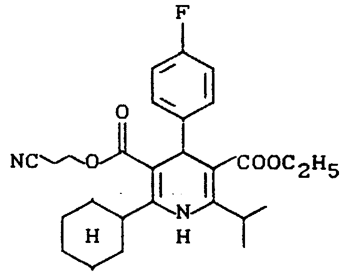

32,9 g (0,1 mol) der Verbindung aus Beispiel 52 und 15,7 g (0,1 mol) 3-Amino-4-methyl-pent-2-en-säureethylester werden in 100 ml Ethanol gelöst und über Nacht am Rückfluß gekocht. Nach Einengen im Vakuum wird der Rückstand in Essigester gelöst, je einmal mit 10 %iger Salzsäure, gesättigter Natriumhydrogencarbonat-Lösung und Wasser gewaschen, die organische Phase über Magnesiumsulfat getrocknet und im Vakuum eingeengt. Der Rückstand wird an einer Säule (Kieselgel 70-230 mesh, mit Dichlormethan) chromatographiert.
- Ausbeute:: 7,8 g (17,6 % der Theorie)

### Beispiel 54

### 2-Cyclohexyl-4-(4-fluorphenyl)-6-isopropyl-pyridin-3,5-dicarbonsäure-3-cyanethylester-5-ethylester

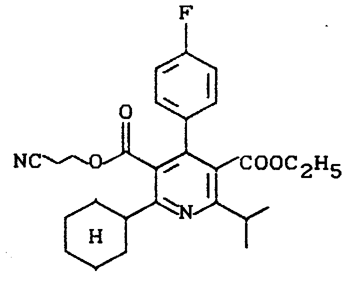

3,53 g (7,55 mmol) der Verbindung aus Beispiel 53 werden analog Beispiel 3 umgesetzt.
- Ausbeute:: 2,96 g (84 % der Theorie)
- ¹H-NMR (CDCl₃):: δ = 0,98 (t, 3H); 1,33 (m, 10H); 1,82 (m, 6H); 2,35 (t, 2H); 2,69 (m, 1H); 3,10 (m, 1H); 4,02 (q, 2H); 4,15 (t, 2H); 7,08 (m, 2H); 7,28 (m, 2H) ppm.

### Beispiel 55

### 2-Cyclohexyl-3,5-dihydroxymethyl-4-(4-fluorphenyl)-6-isopropyl-pyridin

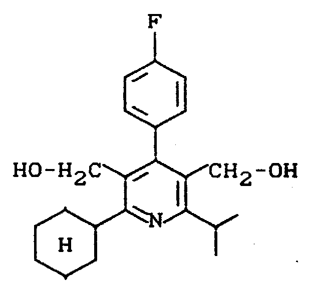

2,5 g (5,35 mmol) der Verbindung aus Beispiel 54 gelöst in 50 ml trockenem Toluol werden bei -78°C unter Stickstoffatmosphäre mit 35,7 ml (53,5 mmol) einer 1,5 molaren Lösung von Diisobutylaluminiumhydrid in Toluol versetzt, 1 Stunde bei -78°C und über Nacht bei Raumtemperatur gerührt. Die Mischung wird unter Eiskühlung mit 20 %iger Kaliumhydroxid-Lösung versetzt und mehrmals mit Toluol extrahiert.

Die organische Phase wird mit Wasser gewaschen, über Magnesiumsulfat getrocknet und im Vakuum eingeengt.
- Ausbeute:: 1,64 g (86 % der Theorie)
- ¹H-NMR (CDCl₃):: δ = 1,20-1,50 (m, 4H); 1,35 (d, 6H); 1,70-1,95 (m, 6H); 3,05 (m, 1H); 3,43 (m, 1H); 4,35 (s, 4H); 7,05-7,25 (m, 4H) ppm.

### Beispiel 56 (EP)

### 2-Cyclohexyl-4-(4-fluorphenyl)-6-isopropyl-3,5-di-(methyl-erythro-(E)-3,5-dihydroxy-hept-6-enoat-7-yl)-pyridin

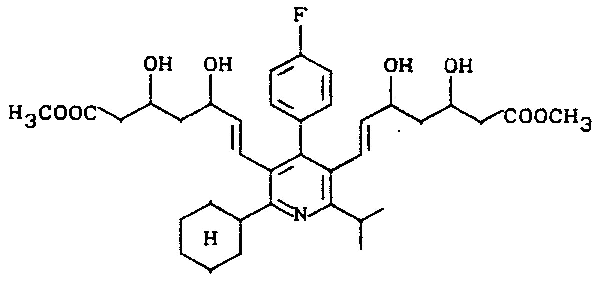

Aus der Verbindung aus Beispiel 55 wurde, in Analogie zu den Reaktionen aus den Beispielen 5, 6, 7, 8, das Beispiel 56 hergestellt.
- ¹H-NMR (CDCl₃):: δ = 1,20-1,85 (m, 20H); 2,41 (m, 4H); 2,90 (m, 1H); 3,28 (m, 1H); 3,70 (s, 6H); 4,08 (m, 2H); 4,30 (m, 2H); 5,25 (dd, 2H); 6,30 (dd, 2H); 7,0 (m, 4H) ppm.

### Beispiel 57

### (E(2)-3-Ethoxycarbonyl-1-(furan-2-yl)-4-methyl-penten-3-on

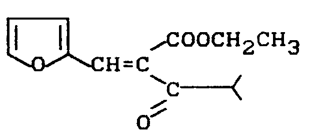

Die Verbindung wurde analog Beispiel 1 aus Furan-2-carbaldehyd hergestellt.
- Ausbeute:: 93 % der Theorie
- Kp 0,5 mbar:: 130°C

### Beispiel 58 (EP)

### 4-(Furan-2-yl)-3,5-bis-[3,5-dihydroxy-6-methoxycarbonyl-hex-1-enyl]-2,6-diisopropyl-pyridin

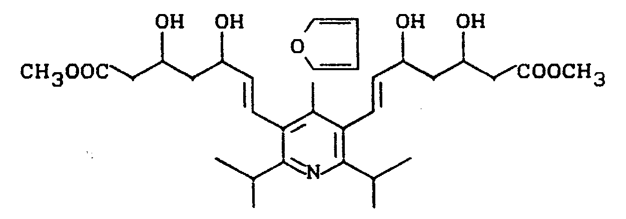

Beispiel 58 wurde aus der Verbindung aus Beispiel 57 analog der Reaktionen der Beispiele 2, 3, 5, 6, 7, 8 und 55 hergestellt.

Farblose Kristalle, Schmelzpunkt 106°C
- ¹H-NMR (CDCl₃):: δ = 1,25 (m, 12H); 1,4 - 1,65 (m, 4H); 2,45 (m, 4H); 2,77 (d, 2H); 3,3 (m, 2H); 3,6 (m, 2H); 3,6 (d, 2H); 3,72 (s, 6H); 4,1 (m, 2H); 4,4 (m, 2H); 5,4 (dd, 2H); 6,12 (m, 1H); 6,4 (m, 1H); 6,5 (d, 2H); 7,45 (m, 1H)

### Beispiel 59

### (E(2)-3-Ethoxycarbonyl-4-methyl-1(thiopen-2-yl)penten-3-on

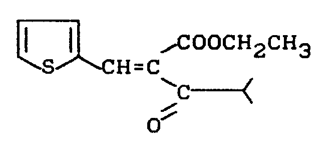

Beispiel 59 wurde analog Beispiel 1 aus Thiopen-2-carbaldehyd hergestellt.
- Ausbeute:: 86 % der Theorie
- Kp 1,5 mbar:: 145°C

### Beispiel 60 (EP)

### 3,5-Bis-[3,5-dihydroxy-6-methoxycarbonyl-hex-1-enyl]-2,6-diisopropyl-4-(thiophen-2-yl)-pyridin

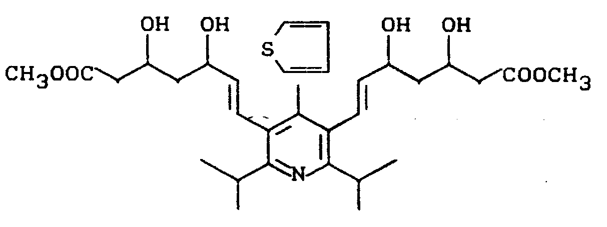

Beispiel 60 wurde aus der Verbindung aus Beispiel 59 analog Beispiele 2, 3, 5, 6, 7, 8 und 27 hergestellt.

Farblose Kristalle, Schmelzpunkt 62°C
- ¹H-NMR (CDCl₃):: δ = 1,25 (m, 12H); 1,35 - 1,6 (m, 4H); 2,45 (m, 4H); 2,6 (s, 2H); 3,3 (m, 2H); 3,5 (d, 2H); 3,72 (s, 6H); 4,1 (m, 2H); 4,35 (m, 2H); 5,48 (dd, 2H); 6,4 (d, 2H); 6,74 (m, 1H); 6,98 (m, 1H); 7,28 (m, 1H)

### Beispiel 61 (EP)

### Di-Natrium-2,6-diisopropyl-4-phenyl-3,5-di-(erythro-(E)-3,5-dihydroxy-hept-6-enoat-7-yl)-pyridin

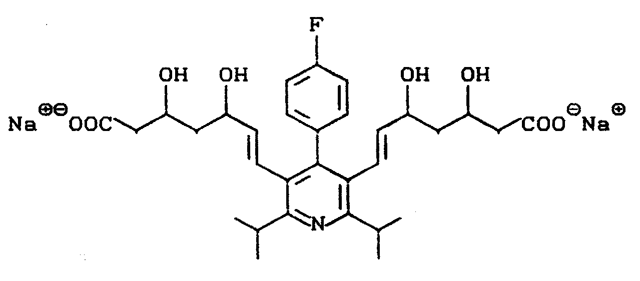

583 mg (1 mmol) der Verbindung aus Beispiel 16 werden in 10 ml Tetrahydrofuran gelöst. Nach Zugabe von 20 ml 0,1 N NaOH läßt man 1 Stunde bei Raumtemperatur stehen, engt im Vakuum ein und lyophilisiert dann die wässrige Lösung.
- Ausbeute:: 605 mg (96,5 % der Theorie).

### Anwendungsbeispiel

### Beispiel 62

Die Enzymaktivitätsbestimmung wurde modifiziert nach G.C. Ness et al., Archives of Biochemistry and Biophysics 197, 493 - 499 (1979) durchgeführt. Männliche Ricoratten (Körpergewicht 300 - 400 g) wurden 11 Tage mit Altrominpulverfutter, dem 40 g Cholestyramin/kg Futter zugesetzt war, behandelt. Nach Dekapitation wurden den Tieren die Lebern entnommen und auf Eis gegeben. Die Lebern wurden zerkleinert und im Potter-Elvejem-Homogenisator 3 mal in 3 Volumen 0,1 m Saccharose, 0,05 m KCl, 0,04 m KₓH_{y} Phosphat, 0,03 m Ethylendiamintetraessigsäure, 0,002 m Dithiothreit (SPE)-Puffer pH 7,2, homogenisiert. Anschließend wurde 15 Minuten bei 15 000* g zentrifugiert und das Sediment verworfen. Der Überstand wurde 75 Minuten bei 100 000 g sedimentiert. Das Pellet wird in 1/4 Volumen SPE-Puffer aufgenommen, nochmals homogenisiert und anschließend erneut 60 Minuten bei 100.000 g zentrifugiert. Das Pellet wird mit der 5-fachen Menge ihres Volumes SPE-Puffer aufgenommen, homogenisiert und bei -78°C eingefroren und gelagert (= Enzymlösung).

Zur Testung wurden die Testverbindungen (oder Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37°C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase, 35 µMol KₓH_{y}-Phosphat pH 7,2, 20 µl Enzympräparation und 56 nMol 3-Hydroxy-3-methyl-glutaryl Coenzym A (Glutaryl-3-¹⁴C) 100 000 dpm.

Man inkubierte 60 Minuten bei 37°C und stoppte die Reaktion durch Zusatz von 300 µl 0,24 m HCl ab. Nach einer Nachinkubation von 60 Minuten bei 37°C wurde der Ansatz zentrifugiert und 600 µl des Überstandes auf eine 0,7 x 4 cm mit Biorex ^{(R)} 5-Chlorid 100-200 mesh (Anionenaustauscher) gefüllte Säule aufgetragen. Es wurde mit 2 ml dest. Wasser nachgewaschen und Durchlauf plus Waschwasser mit 3 ml Aquasol versetzt und im LKB-Scintillationszähler gezählt. IC₅₀-Werte wurden durch Auftrag der prozentualen Hemmung gegen die Konzentration der Verbindung im Test durch Intrapolation bestimmt. Zur Bestimmung der relativen inhibitorischen Potenz wurde der IC₅₀-Wert der Referenzsubstanz Mevinolin als 100 gesetzt und mit dem simultan bestimmten IC₅₀-Wert der Testverbindung verglichen.

### Beispiel 63

Die subchronische Wirkung der disubstituierten Pyridine auf die Blutcholesterinwerte von Hunden wurde in mehrwöchigen Fütterungsexperimenten geprüft. Dazu wurde die zu untersuchende Substanz über einen mehrwöchigen Zeitraum einmal täglich in einer Kapsel gesunden Beagle Hunden zusammen mit dem Futter p.o. gegeben. Dem Futter war außerdem während der gesamten Versuchsperiode, d.h. vor, während und nach der Applikationsperiode der zu untersuchenden Substanz Colestyramin (4 g/100 g Futter) als Gallensäuresequestrant beigemischt. Zweimal wöchentlich wurde den Hunden venöses Blut abgenommen und das Serumcholesterin enzymatisch bestimmt. Die Serumcholesterinwerte während der Applikationsperiode wurden mit den Serumcholesterinwerten vor der Applikationsperiode (Kontrollen) verglichen.

So ergab sich z.B. für das Na-Salz von Beispiel 8 (2,6-Diisopropyl-4-(4-fluorphenyl)-3,5-di-(natrium-erythro-(E)-3,5-dihydroxy-hept-6-enoat-7-yl)-pyridin) nach 2-wöchiger Applikation von täglich 8 mg/kg p.o. eine Senkung des Serumcholesterins um 22,4 %.

## Patentansprüche (Patentansprüche für folgende(n) Vertragsstaat(en): AT, BE, CH, DE, FR, GB, GR, IT, LI, NL, SE)

1. Disubstituierte Pyridine der Formeln 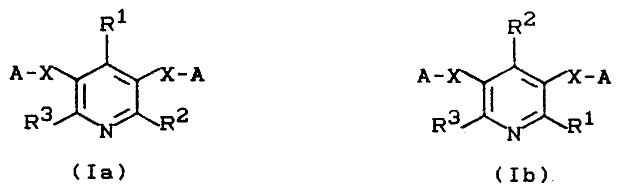 in welchen
R¹
- Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder verschieden substituiert sein kann durch Fluor, Chlor, Brom, C₁-C₆-Alkyl, C₁-C₆-Alkoxy, Phenyl, Phenoxy, Trifluormethyl, Trifluormethoxy oder C₁-C₆-Alkoxycarbonyl, oder
- Phenyl oder Naphthyl bedeutet, das bis zu 4-fach gleich oder verschieden substituiert sein kann durch C₁-C₆-Alkyl, C₁-C₆-Alkoxy, C₁-C₆-Alkylthio, C₁-C₆-Alkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, C₁-C₆-Alkoxycarbonyl oder durch eine Gruppe der Formel -NR⁴R⁵,
wobei
R⁴ und R⁵ gleich oder verschieden sind und C₁-C₆-Alkyl, Phenyl, Benzyl, Acetyl, Benzoyl, Phenylsulfonyl oder C₁-C₆-Alkylsulfonyl bedeuten,
R²
- Cyclopropyl, Cyclopentyl oder Cyclohexyl bedeutet, oder
- C₁-C₆-Alkyl bedeutet, das substituiert sein kann durch Fluor, Chlor, Brom, Cyano, C₁-C₆-Alkoxy, C₁-C₆-Alkylthio, C₁-C₆-Alkylsulfonyl, Phenyl, Trifluormethyl, Trifluormethoxy, Trifluormethylsulfonyl, C₁-C₆-Alkoxycarbonyl, Benzoyl, C₁-C₆-Alkylcarbonyl, durch eine Gruppe der Formel -NR⁴R⁵,
worin
R⁴ und R⁵ die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, C₁-C₆-Alkyl, C₁-C₆-Alkoxy, Trifluormethyl, oder Trifluormethoxy substituiert sein können,
R³
- Wasserstoff, oder
- Cyclopropyl, Cyclopentyl oder Cyclohexyl bedeutet, oder
- C₁-C₆-Alkyl bedeutet, das substituiert sein kann durch Fluor, Chlor, Brom, Cyano, C₁-C₆-Alkoxy, C₁-C₆-Alkylthio, C₁-C₆-Alkylsulfonyl, Trifluormethyl, Trifluormethoxy, Trifluormethylsulfonyl, C₁-C₆-Alkoxycarbonyl, Benzoyl, C₁-C₆-Alkylcarbonyl, durch eine Gruppe der Formel -NR₄R₅,
worin
R⁵ und R⁶ die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl-und Arylreste bis zu 2-fach gleich oder verschieden durch Fluor, Chlor, Brom, C₁-C₆-Alkyl, C₁-C₆-Alkoxy, Trifluormethyl, oder Trifluormethoxy substituiert sein können,
- Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder verschieden substituiert sein kann durch Fluor, Chlor, Brom, C₁-C₆-Alkyl, C₁-C₆-Alkoxy, Phenyl, Phenoxy, Trifluormethyl, Trifluormethoxy oder C₁-C₆-Alkoxycarbonyl, oder
- Phenyl oder Naphthyl bedeutet, das bis zu 4-fach gleich oder verschieden substituiert sein kann durch C₁-C₆-Alkyl, C₁-C₆-Alkoxy, C₁-C₆-Alkylthio, C₁-C₆-Alkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, C₁-C₆-Alkoxycarbonyl oder durch eine Gruppe der Formel -NR⁵R⁶,
wobei
R⁵ und R⁶ die oben angegebene Bedeutung haben,
X
- eine Gruppe der Formel -CH=CH- bedeutet
und
A
- eine Gruppe der Formel 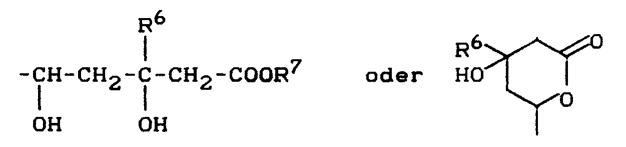 bedeutet,
worin
R⁶
- Wasserstoff oder C₁-C₆-Alkyl bedeutet,
und
R⁷
- C₁-C₆-Alkyl, Phenyl oder Benzyl bedeutet, oder
- ein physiologisch verträgliches Kation bedeutet.

2. Disubstituierte Pyridine nach Anspruch 1
worin
R¹
- Pyridyl, Pyrimidyl, Chinolyl oder Isochinolyl bedeutet, das durch Fluor, Chlor, Methyl, Methoxy oder Trifluormethyl substituiert sein kann, oder
- Phenyl bedeutet, das bis zu 3-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl,
R²
- Cyclopropyl, Cyclopentyl oder Cyclohexyl bedeutet, oder
- Methyl, Ethyl, Propyl, Isopropyl, Butyl, sec.-Butyl oder tert.-Butyl bedeutet, das substituiert sein kann durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,
R³
- Wasserstoff, Cyclopropyl, Cyclopentyl oder Cyclohexyl bedeutet, oder
- Methyl, Ethyl, Propyl, Isopropyl, Butyl, Isobutyl, tert.Butyl, Pentyl, Isopentyl, Hexyl oder Isohexyl bedeutet, das substituiert sein kann durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.-Butoxycarbonyl, Benzoyl, Acetyl, Ethylcarbonyl, oder durch eine Gruppe -NR⁴R⁵,
wobei
R⁴ und R⁵ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,
oder durch Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein können, oder
- Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl bedeutet, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.-Butoxycarbonyl substituiert sein können, oder
- Phenyl bedeutet, das bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.-Butoxycarbonyl oder durch eine Gruppe -NR⁴R⁵ substituiert sein kann,
wobei
R⁴ und R⁵ die oben angegebene Bedeutung haben,
X
- eine Gruppe der Formel -CH=CH- bedeutet
und
A
- eine Gruppe der Formel 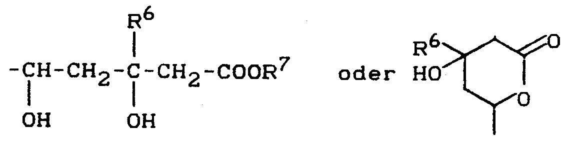 bedeutet,
worin
R⁶
- Wasserstoff, Methyl, Ethyl, Propyl, Isopropyl, Butyl, Isobutyl oder tert.Butyl bedeutet
und
R⁷
- Wasserstoff, Methyl, Ethyl, Propyl, Isopropyl, Butyl, Isobutyl, tert.Butyl oder Benzyl bedeutet, oder
- ein Natrium-, Kalium-, Calcium- oder Magnesium- oder Ammoniumion bedeutet.

3. Disubstituierte Pyridine nach Anspruch 1
wobei
R¹
- Phenyl bedeutet, das bis zu 2-fach gleich oder verschieden durch Methyl, Ethyl, Propyl, Isopropyl, Phenoxy und/oder Fluor substituiert sein kann,
R²
- für Cyclopropyl oder Cyclohexyl steht oder Methyl, Ethyl, Propyl, Isopropyl, Butyl, Isobutyl oder tert.Butyl bedeutet, das durch Fluor, Chlor, Methoxy, Phenyl oder Phenoxy substituiert sein kann,
R³
- Wasserstoff, Cyclopropyl, Cyclopentyl oder Cyclohexyl oder Phenyl bedeutet, oder
- Methyl, Ethyl, Propyl, Isopropyl, Butyl oder Benzyl bedeutet,
X
- eine Gruppe der Formel 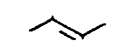 (E-konfiguriert) bedeutet
und
A
- eine Gruppe der Formel 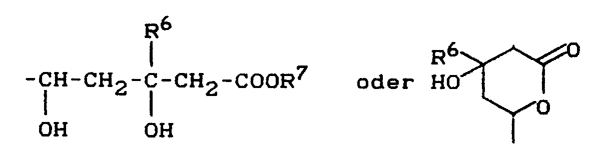 bedeutet,
worin
R⁶
- Wasserstoff bedeutet
und
R⁷
- Wasserstoff, Methyl oder Ethyl bedeutet, oder
- ein Natrium- oder Kaliumkation bedeutet.

4. Disubstituierte Pyridine nach den Ansprüchen 1 bis 3 zur therapeutischen Behandlung.

5. Verfahren zur Herstellung von disubstituierten Pyridinen nach Anspruch 1, dadurch gekennzeichnet, daß man Ketone der allgemeinen Formel (VIII) 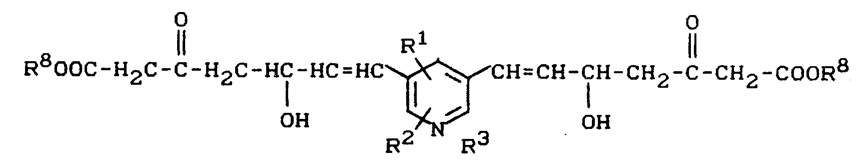 in welcher
R¹, R² und R³ die oben angegebene Bedeutung haben,
und
R⁸
- für C₁-C₁₂-Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
und gegebenenfalls Isomeren trennt.

6. Ketone der Formel 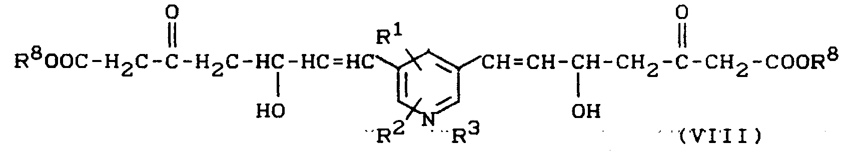 in welcher
R¹, R², R³ die oben in Anspruch 1 angegebene Bedeutung haben
und
R⁸ für C₁-C₁₂-Alkyl steht.

7. Verfahren nach Anspruch 8, dadurch gekennzeichnet, daß man die Umsetzung im Temperaturbereich von - 80°C bis +50°C durchführt.

8. Arzneimittel, enthaltend disubstituierte Pyridine nach den Ansprüchen 1 bis 3.

9. Verwendung von disubstituierten Pyridinen nach den Ansprüchen 1 bis 3 zur Herstellung von Arzneimitteln.

10. Verwendung nach Anspruch 13 zur Herstellung von Arzneimitteln zur Behandlung von Lipoproteinämie und Arteriosklerose.

11. Arzneimittel nach Anspruch 10, enthaltend 0,5 bis 98,5 Gew.-% an disubstituierten Pyridinen.

## Patentansprüche (Patentansprüche für folgende(n) Vertragsstaat(en): ES)

1. Verfahren zur Herstellung von disubstituierten Pyridinen der Formeln 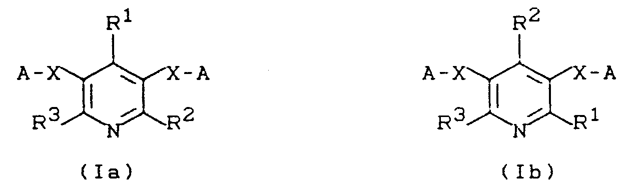 in welchen
R¹
- Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder verschieden substituiert sein kann durch Fluor, Chlor, Brom, C₁-C₆-Alkyl, C₁-C₆-Alkoxy, Phenyl, Phenoxy, Trifluormethyl, Trifluormethoxy oder C₁-C₆-Alkoxycarbonyl, oder
- Phenyl oder Naphthyl bedeutet, das bis zu 4-fach gleich oder verschieden substituiert sein kann durch C₁-C₆-Alkyl, C₁-C₆-Alkoxy, C₁-C₆-Alkylthio, C₁-C₆-Alkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, C₁-C₆-Alkoxycarbonyl oder durch eine Gruppe der Formel -NR⁴R⁵,
wobei
R⁴ und R⁵ gleich oder verschieden sind und C₁-C₆-Alkyl, Phenyl, Benzyl, Acetyl, Benzoyl, Phenylsulfonyl oder C₁-C₆-Alkylsulfonyl bedeuten,
R²
- Cyclopropyl, Cyclopentyl oder Cyclohexyl bedeutet, oder
- C₁-C₆-Alkyl bedeutet, das substituiert sein kann durch Fluor, Chlor, Brom, Cyano, C₁-C₆-Alkoxy, C₁-C₆-Alkylthio, C₁-C₆-Alkylsulfonyl, Phenyl, Trifluormethyl, Trifluormethoxy, Trifluormethylsulfonyl, C₁-C₆-Alkoxycarbonyl, Benzoyl, C₁-C₆-Alkylcarbonyl, durch eine Gruppe der Formel -NR⁴R⁵,
worin
R⁴ und R⁵ die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, C₁-C₆-Alkyl, C₁-C₆-Alkoxy, Trifluormethyl, oder Trifluormethoxy substituiert sein können,
R³
- Wasserstoff, oder
- Cyclopropyl, Cyclopentyl oder Cyclohexyl bedeutet, oder
- C₁-C₆-Alkyl bedeutet, das substituiert sein kann durch Fluor, Chlor, Brom, Cyano, C₁-C₆-Alkoxy, C₁-C₆-Alkylthio, C₁-C₆-Alkylsulfonyl, Trifluormethyl, Trifluormethoxy, Trifluormethylsulfonyl, C₁-C₆-Alkoxycarbonyl, Benzoyl, C₁-C₆-Alkylcarbonyl, durch eine Gruppe der Formel -NR₄R₅,
worin
R⁵ und R⁶ die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl-und Arylreste bis zu 2-fach gleich oder verschieden durch Fluor, Chlor, Brom, C₁-C₆-Alkyl, C₁-C₆-Alkoxy, Trifluormethyl, oder Trifluormethoxy substituiert sein können,
- Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder verschieden substituiert sein kann durch Fluor, Chlor, Brom, C₁-C₆-Alkyl, C₁-C₆-Alkoxy, Phenyl, Phenoxy, Trifluormethyl, Trifluormethoxy oder C₁-C₆-Alkoxycarbonyl, oder
- Phenyl oder Naphthyl bedeutet, das bis zu 4-fach gleich oder verschieden substituiert sein kann durch C₁-C₆-Alkyl, C₁-C₆-Alkoxy, C₁-C₆-Alkylthio, C₁-C₆-Alkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, C₁-C₆-Alkoxycarbonyl oder durch eine Gruppe der Formel -NR⁵R⁶,
wobei
R⁵ und R⁶ die oben angegebene Bedeutung haben,
X
- eine Gruppe der Formel -CH=CH- bedeutet
und
A
- eine Gruppe der Formel 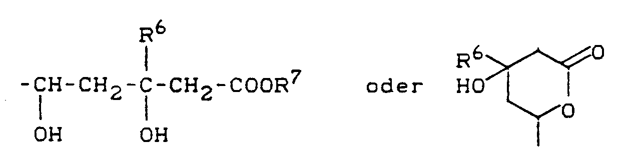 bedeutet,
worin
R⁶
- Wasserstoff oder C₁-C₆-Alkyl bedeutet,
und
R⁷
- C₁-C₆-Alkyl, Phenyl oder Benzyl bedeutet, oder
- ein physiologisch verträgliches Kation bedeutet,
dadurch gekennzeichnet, daß man
Ketone der allgemeinen Formel (VIII) 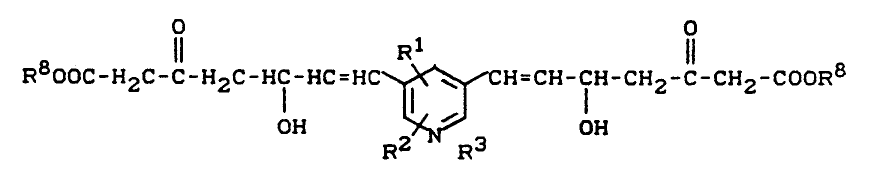 in welcher
R¹, R² und R³ die oben angegebene Bedeutung haben,
und
R⁸
- für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
und gegebenenfalls Isomeren trennt.

2. Verfahren zu Herstellung von Ketonen der Formel 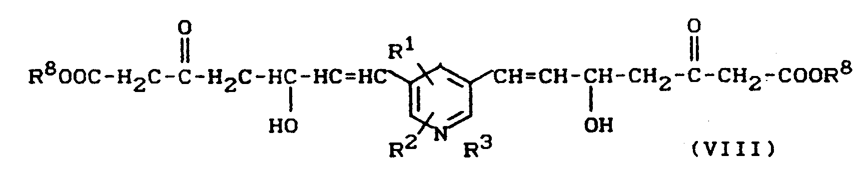 wobei
R¹-R³ die in Anspruch 1 angegebene Bedeutung haben
und
R⁸ für Alkyl steht
dadurch gekennzeichnet, daß man
Aldehyde der allgemeinen Formel (IX) 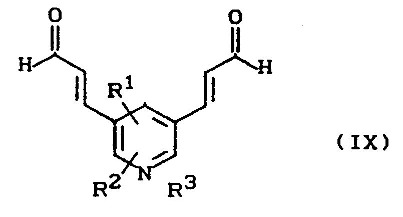 in welcher
R¹, R² und R³ die oben angegebene Bedeutung haben,
in inerten Lösemitteln mit Acetessigester der allgemeinen Formel (X) 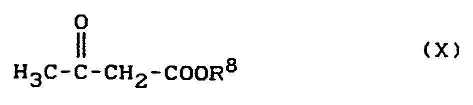 in welcher
R⁸ die oben angegebene Bedeutung hat,
in Anwesenheit von Basen umsetzt.

3. Verwendung Von disubstituierten Pyridinen der allgemeinen Formeln I a, b 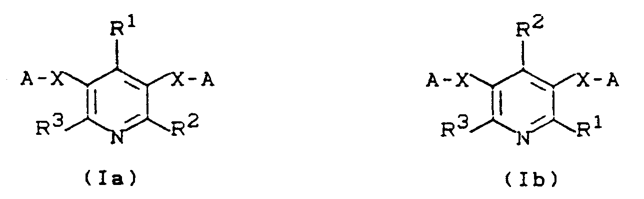 worin
R¹ - R³, A und X die in Anspruch 1 angegebene Bedeutung haben zur Herstellung von Arzneimitteln.

## Claims (Claims for the following Contracting State(s): AT, BE, CH, DE, FR, GB, GR, IT, LI, NL, SE)

1. Disubstituted pyridines of the formulae 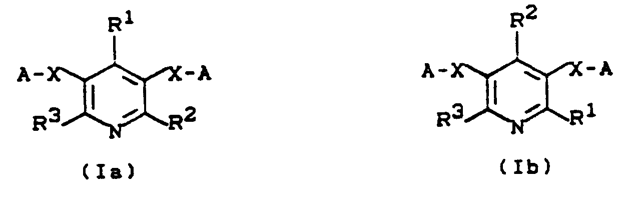 in which
R¹
- denotes thienyl, furyl, thiazolyl, isothiazolyl, oxazolyl, isoxazolyl, pyridyl, pyrimidyl, pyrazinyl, pyridazinyl, indolyl, isoindolyl, quinolyl, isoquinolyl, phthalazinyl, quinoxalinyl, quinazolinyl, cinnolinyl, benzothiazolyl, benzoxazolyl or benzimidazolyl which can be substituted identically or differently up to 2 times by fluorine, chlorine, bromine, C₁-C₆-alkyl, C₁-C₆-alkoxy, phenyl, phenoxy, trifluoromethyl, trifluoromethoxy or C₁-C₆-alkoxycarbonyl, or
- denotes phenyl or naphthyl which can be substituted identically or differently up to 4 times by C₁-C₆-alkyl, C₁-C₆-alkoxy, C₁-C₆-alkylthio, C₁-C₆-alkylsulphonyl, phenyl, phenyloxy, phenylthio, phenylsulphonyl, benzyl, benzyloxy, benzylthio, benzylsulphonyl, phenethyl, phenylethoxy, phenylethylthio, phenylethylsulphonyl, fluorine, chlorine, bromine, cyano, trifluoromethyl, trifluoromethoxy, trifluoromethylthio, C₁-C₆-alkoxycarbonyl or by a group of the formula -NR⁴R⁵,
where
R⁴ and R⁵ are identical or different and denote C₁-C₆-alkyl, phenyl, benzyl, acetyl, benzoyl, phenylsulphonyl or C₁-C₆-alkylsulphonyl,
R²
- denotes cyclopropyl, cyclopentyl or cyclohexyl, or
- denotes C₁-C₆-alkyl which can be substituted by fluorine, chlorine, bromine, cyano, C₁-C₆-alkoxy, C₁-C₆-alkylthio, C₁-C₆-alkylsulphonyl, phenyl, trifluoromethyl, trifluoromethoxy, trifluoromethylsulphonyl, C₁-C₆-alkoxycarbonyl, benzoyl, C₁-C₆-alkylcarbonyl, by a group of the formula -NR⁴R⁵,
in which
R⁴ and R⁵ have the abovementioned meaning,
or by pyridyl, pyrimidyl, pyrazinyl, pyridazinyl, quinolyl, isoquinolyl, pyrrolyl, indolyl, thienyl, furyl, imidazolyl, oxazolyl, thiazolyl, phenyl, phenoxy, phenylthio, phenylsulphonyl, benzyloxy, benzylthio, benzylsulphonyl, phenylethoxy, phenylethylthio or phenylethylsulphonyl, where the said heteroaryl and aryl radicals can be substituted identically or differently up to 2 times by fluorine, chlorine, bromine, C₁-C₆-alkyl, C₁-C₆-alkoxy, trifluoromethyl or trifluoromethoxy,
R³
- denotes hydrogen, or
- cyclopropyl, cyclopentyl or cyclohexyl, or
- denotes C₁-C₆-alkyl which can be substituted by fluorine, chlorine, bromine, cyano, C₁-C₆-alkoxy, C₁-C₆-alkylthio, C₁-C₆-alkylsulphonyl, trifluoromethyl, trifluoromethoxy, trifluoromethylsulphonyl, C₁-C₆-alkoxycarbonyl, benzoyl, C₁-C₆-alkylcarbonyl, by a group of the formula -NR₄R₅,
in which
R⁵ and R⁶ have the abovementioned meaning,
or by pyridyl, pyrimidyl, pyrazinyl, pyridazinyl, quinolyl, isoquinolyl, pyrrolyl, indolyl, thienyl, furyl, imidazolyl, oxazolyl, thiazolyl, phenyl, phenoxy, phenylthio, phenylsulphonyl, benzyloxy, benzylthio, benzylsulphonyl, phenylethoxy, phenylethylthio or phenylethylsulphonyl, where the said heteroaryl and aryl radicals can be substituted identically or differently up to 2 times by fluorine, chlorine, bromine, C₁-C₆-alkyl, C₁-C₆-alkoxy, trifluoromethyl or trifluoromethoxy,
- denotes thienyl, furyl, thiazolyl, isothiazolyl, oxazolyl, isoxazolyl, pyridyl, pyrimidyl, pyrazinyl, pyridazinyl, indolyl, isoindolyl, quinolyl, isoquinolyl, phthalazinyl, quinoxalinyl, quinazolinyl, cinnolinyl, benzothiazolyl, benzoxazolyl or benzimidazolyl which can be substituted identically or differently up to 2 times by fluorine, chlorine, bromine, C₁-C₆-alkyl, C₁-C₆-alkoxy, phenyl, phenoxy, trifluoromethyl, trifluoromethoxy or C₁-C₆-alkoxycarbonyl, or
- denotes phenyl or naphthyl which can be substituted identically or differently up to 4 times by C₁-C₆-alkyl, C₁-C₆-alkoxy, C₁-C₆-alkylthio, C₁-C₆-alkylsulphonyl, phenyl, phenyloxy, phenylthio, phenylsulphonyl, benzyl, benzyloxy, benzylthio, benzylsulphonyl, phenethyl, phenylethoxy, phenylethylthio, phenylethylsulphonyl, fluorine, chlorine, bromine, cyano, trifluoromethyl, trifluoromethoxy, trifluoromethylthio, C₁-C₆-alkoxycarbonyl or by a group of the formula -NR⁵R⁶,
where
R⁵ and R⁶ have the abovementioned meaning,
X
- denotes a group of the formula -CH=CH-
and
A
- denotes a group of the formula 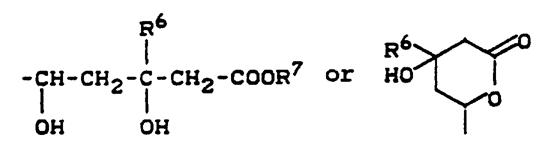 in which
R⁶
- denotes hydrogen or C₁-C₆-alkyl,
and
R⁷
- denotes C₁-C₆-alkyl, phenyl or benzyl, or
- denotes a physiologically tolerated cation.

2. Disubstituted pyridines according to Claim 1,
in which
R¹
- denotes pyridyl, pyrimidyl, quinolyl or isoquinolyl which can be substituted by fluorine, chlorine, methyl, methoxy or trifluoromethyl, or
- denotes phenyl which can be substituted identically or differently up to 3 times by methyl, ethyl, propyl, isopropyl, butyl, isobutyl, tert-butyl, methoxy, ethoxy, propoxy, isopropoxy, butoxy, isobutoxy, tert-butoxy, methylthio, ethylthio, propylthio, isopropylthio, methylsulphonyl, ethylsulphonyl, propylsulphonyl, isopropylsulphonyl, phenyl, phenoxy, benzyl, benzyloxy, fluorine, chlorine, bromine, cyano, trifluoromethyl, trifluoromethoxy, methoxycarbonyl, ethoxycarbonyl, propoxycarbonyl, isopropoxycarbonyl, butoxycarbonyl, isobutoxycarbonyl or tert-butoxycarbonyl,
R²
- denotes cyclopropyl, cyclopentyl or cyclohexyl, or
- denotes methyl, ethyl, propyl, isopropyl, butyl, sec-butyl or tert-butyl which can be substituted by fluorine, chlorine, bromine, cyano, methoxy, ethoxy, propoxy, isopropoxy, butoxy, sec-butoxy, tert-butoxy, methylthio, ethylthio, propylthio, isopropylthio, methylsulphonyl, ethylsulphonyl, propylsulphonyl, isopropylsulphonyl, trifluoromethyl, trifluoromethoxy, methoxycarbonyl, ethoxycarbonyl, butoxycarbonyl, isobutoxycarbonyl, tert-butoxycarbonyl, benzoyl, acetyl, pyridyl, pyrimidyl, thienyl, furyl, phenyl, phenoxy, phenylthio, phenylsulphonyl, benzyloxy, benzylthio or benzylsulphonyl,
R³
- denotes hydrogen, cyclopropyl, cyclopentyl or cyclohexyl, or
- denotes methyl, ethyl, propyl, isopropyl, butyl, isobutyl, tert-butyl, pentyl, isopentyl, hexyl or isohexyl which can be substituted by fluorine, chlorine, bromine, cyano, methoxy, ethoxy, propoxy, isopropoxy, butoxy, isobutoxy, tert-butoxy, methylthio, ethylthio, propylthio, isopropylthio, butylthio, isobutylthio, tert-butylthio, methylsulphonyl, ethylsulphonyl, propylsulphonyl, isopropylsulphonyl, butylsulphonyl, isobutylsulphonyl, tert-butylsulphonyl, trifluoromethyl, trifluoromethoxy, methoxycarbonyl, ethoxycarbonyl, propoxycarbonyl, isopropoxycarbonyl, butoxycarbonyl, isobutoxycarbonyl, tert-butoxycarbonyl, benzoyl, acetyl, ethylcarbonyl, or by a group -NR⁴R⁵,
where
R⁴ and R⁵ are identical or different and denote methyl, ethyl, propyl, isopropyl, butyl, isobutyl, tert-butyl, phenyl, benzyl, acetyl, methylsulphonyl, ethylsulphonyl, propylsulphonyl, isopropylsulphonyl or phenylsulphonyl,
or by pyridyl, pyrimidyl, pyrazinyl, pyridazinyl, quinolyl, isoquinolyl, thienyl, furyl, phenyl, phenoxy, phenylthio, phenylsulphonyl, benzyloxy, benzylthio or benzylsulphonyl, where the said heteroaryl and aryl radicals can be substituted by fluorine, chlorine, methyl, ethyl, propyl, isopropyl, isobutyl, tert-butyl, methoxy, ethoxy, propoxy, isopropoxy, butoxy, isobutoxy, tert-butoxy, trifluoromethyl or trifluoromethoxy, or
- denotes thienyl, furyl, pyridyl, pyrimidyl, pyrazinyl, pyridazinyl, oxazolyl, isooxazolyl, imidazolyl, pyrazolyl, thiazolyl, isothiazolyl, quinolyl, isoquinolyl, benzoxazolyl, benzimidazolyl or benzothiazolyl, where the said radicals can be substituted by fluorine, chlorine, methyl, ethyl, propyl, isopropyl, butyl, isobutyl, tert-butyl, methoxy, ethoxy, propoxy, isopropoxy, butoxy, isobutoxy, tert-butoxy, phenyl, phenoxy, trifluoromethyl, trifluoromethoxy, methoxycarbonyl, ethoxycarbonyl, isopropoxycarbonyl, propoxycarbonyl, butoxycarbonyl, isobutoxycarbonyl or tert-butoxycarbonyl, or
- denotes phenyl which can be substituted identically or differently up to 3 times by methyl, ethyl, propyl, isopropyl, butyl, isobutyl, tert-butyl, pentyl, isopentyl, hexyl, isohexyl, methoxy, ethoxy, propoxy, isopropoxy, butoxy, isobutoxy, tert-butoxy, methylthio, ethylthio, propylthio, isopropylthio, butylthio, isobutylthio, tert-butylthio, methylsulphonyl, ethylsulphonyl, propylsulphonyl, isopropylsulphonyl, butylsulphonyl, isobutylsulphonyl, tert-butylsulphonyl, phenyl, phenoxy, phenylthio, phenylsulphonyl, benzyl, benzyloxy, benzylthio, benzylsulphonyl, fluorine, chlorine, bromine, cyano, trifluoromethyl, trifluoromethoxy, trifluoromethylthio, methoxycarbonyl, ethoxycarbonyl, propoxycarbonyl, isopropoxycarbonyl, butoxycarbonyl, isobutoxycarbonyl, tert-butoxycarbonyl or by a group -NR⁴R⁵,
where
R⁴ and R⁵ have the abovementioned meaning,
X
- denotes a group of the formula -CH=CH-,
and
A
- denotes a group of the formula 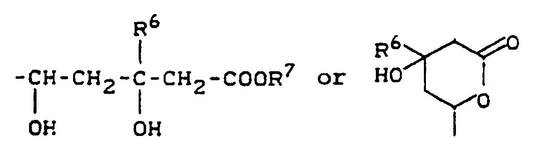 in which
R⁶
- denotes hydrogen, methyl, ethyl, propyl, isopropyl, butyl, isobutyl or tert-butyl,
and
R⁷
- denotes hydrogen, methyl, ethyl, propyl, isopropyl, butyl, isobutyl, tert-butyl or benzyl, or
- denotes a sodium, potassium, calcium or magnesium or ammonium ion.

3. Disubstituted pyridines according to Claim 1,
where
R¹
- denotes phenyl which can be substituted identically or differently up to 2 times by methyl, ethyl, propyl, isopropyl, phenoxy and/or fluorine,
R²
- represents cyclopropyl or cyclohexyl, or denotes methyl, ethyl, propyl, isopropyl, butyl, isobutyl or tert-butyl which can be substituted by fluorine, chlorine, methoxy, phenyl or phenoxy,
R³
- denotes hydrogen, cyclopropyl, cyclopentyl or cyclohexyl or phenyl, or
- denotes methyl, ethyl, propyl, isopropyl, butyl or benzyl,
X
- denotes a group of the formula 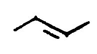 (E-configured)
and
A
- denotes a group of the formula 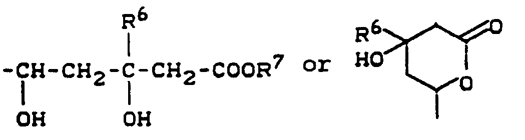 in which
R⁶
- denotes hydrogen
and
R⁷
- denotes hydrogen, methyl or ethyl, or
- denotes a sodium or potassium cation.

4. Disubstituted pyridines according to Claims 1 to 3 for therapeutic treatment.

5. Process for preparing disubstituted pyridines according to Claim 1, characterized in that ketones of the general formula (VIII) 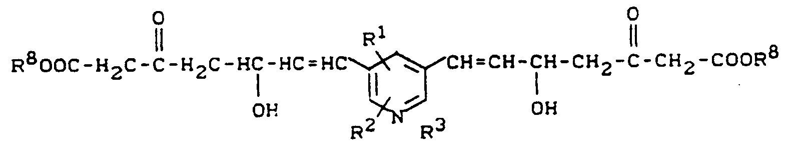 in which
R¹, R² and R³ have the abovementioned meaning,
and
R⁸
- represents C₁-C₁₂-alkyl,
are reduced,
the esters are hydrolysed when preparing the acids,
the carboxylic acids are cyclized when preparing the lactones,
either the esters or the lactones are hydrolysed when preparing the salts,
and isomers are optionally resolved.

6. Ketones of the formula 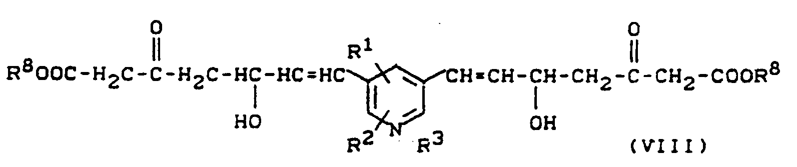 in which
R¹, R² and R³ have the meaning given above in Claim 1,
and
R⁸ represents C₁-C₁₂-alkyl.

7. Process according to Claim 8, characterized in that the reaction is carried out in a temperature range of from -80°C to +50°C.

8. Medicament containing disubstituted pyridines according to Claims 1 to 3.

9. Use of disubstituted pyridines according to Claims 1 to 3 for preparing medicaments.

10. Use according to Claim 13 for preparing medicaments for treating lipoproteinaemia and arteriosclerosis.

11. Medicament according to Claim 10, containing 0.5 to 98.5% by weight of disubstituted pyridines.

## Claims (Claims for the following Contracting State(s): ES)

1. Process for preparing disubstituted pyridines of the formulae 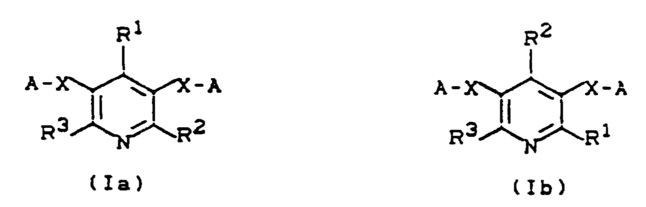 in which
R¹
- denotes thienyl, furyl, thiazolyl, isothiazolyl, oxazolyl, isoxazolyl, pyridyl, pyrimidyl, pyrazinyl, pyridazinyl, indolyl, isoindolyl, quinolyl, isoquinolyl, phthalazinyl, quinoxalinyl, quinazolinyl, cinnolinyl, benzothiazolyl, benzoxazolyl or benzimidazolyl which can be substituted identically or differently up to 2 times by fluorine, chlorine, bromine, C₁-C₆-alkyl, C₁-C₆-alkoxy, phenyl, phenoxy, trifluoromethyl, trifluoromethoxy or C₁-C₆-alkoxycarbonyl, or
- denotes phenyl or naphthyl which can be substituted identically or differently up to 4 times by C₁-C₆-alkyl, C₁-C₆-alkoxy, C₁-C₆-alkylthio, C₁-C₆-alkylsulphonyl, phenyl, phenyloxy, phenylthio, phenylsulphonyl, benzyl, benzyloxy, benzylthio, benzylsulphonyl, phenethyl, phenylethoxy, phenylethylthio, phenylethylsulphonyl, fluorine, chlorine, bromine, cyano, trifluoromethyl, trifluoromethoxy, trifluoromethylthio, C₁-C₆-alkoxycarbonyl or by a group of the formula -NR⁴R⁵,
where
R⁴ and R⁵ are identical or different and denote C₁-C₆-alkyl, phenyl, benzyl, acetyl, benzoyl, phenylsulphonyl or C₁-C₆-alkylsulphonyl,
R²
- denotes cyclopropyl, cyclopentyl or cyclohexyl, or
- denotes C₁-C₆-alkyl which can be substituted by fluorine, chlorine, bromine, cyano, C₁-C₆-alkoxy, C₁-C₆-alkylthio, C₁-C₆-alkylsulphonyl, phenyl, trifluoromethyl, trifluoromethoxy, trifluoromethylsulphonyl, C₁-C₆-alkoxycarbonyl, benzoyl, C₁-C₆-alkylcarbonyl, by a group of the formula -NR⁴R⁵,
in which
R⁴ and R⁵ have the abovementioned meaning,
or by pyridyl, pyrimidyl, pyrazinyl, pyridazinyl, quinolyl, isoquinolyl, pyrrolyl, indolyl, thienyl, furyl, imidazolyl, oxazolyl, thiazolyl, phenyl, phenoxy, phenylthio, phenylsulphonyl, benzyloxy, benzylthio, benzylsulphonyl, phenylethoxy, phenylethylthio or phenylethylsulphonyl, where the said heteroaryl and aryl radicals can be substituted identically or differently up to 2 times by fluorine, chlorine, bromine, C₁-C₆-alkyl, C₁-C₆-alkoxy, trifluoromethyl or trifluoromethoxy,
R³
- denotes hydrogen, or
- cyclopropyl, cyclopentyl or cyclohexyl, or
- denotes C₁-C₆-alkyl which can be substituted by fluorine, chlorine, bromine, cyano, C₁-C₆-alkoxy, C₁-C₆-alkylthio, C₁-C₆-alkylsulphonyl, trifluoromethyl, trifluoromethoxy, trifluoromethylsulphonyl, C₁-C₆-alkoxycarbonyl, benzoyl, C₁-C₆-alkylcarbonyl, by a group of the formula -NR₄R₅,
in which
R⁵ and R⁶ have the abovementioned meaning,
or by pyridyl, pyrimidyl, pyrazinyl, pyridazinyl, quinolyl, isoquinolyl, pyrrolyl, indolyl, thienyl, furyl, imidazolyl, oxazolyl, thiazolyl, phenyl, phenoxy, phenylthio, phenylsulphonyl, benzyloxy, benzylthio, benzylsulphonyl, phenylethoxy, phenylethylthio or phenylethylsulphonyl, where the said heteroaryl and aryl radicals can be substituted identically or differently up to 2 times by fluorine, chlorine, bromine, C₁-C₆-alkyl, C₁-C₆-alkoxy, trifluoromethyl or trifluoromethoxy,
- denotes thienyl, furyl, thiazolyl, isothiazolyl, oxazolyl, isoxazolyl, pyridyl, pyrimidyl, pyrazinyl, pyridazinyl, indolyl, isoindolyl, quinolyl, isoquinolyl, phthalazinyl, quinoxalinyl, quinazolinyl, cinnolinyl, benzothiazolyl, benzoxazolyl or benzimidazolyl which can be substituted identically or differently up to 2 times by fluorine, chlorine, bromine, C₁-C₆-alkyl, C₁-C₆-alkoxy, phenyl, phenoxy, trifluoromethyl, trifluoromethoxy or C₁-C₆-alkoxycarbonyl, or
- denotes phenyl or naphthyl which can be substituted identically or differently up to 4 times by C₁-C₆-alkyl, C₁-C₆-alkoxy, C₁-C₆-alkylthio, C₁-C₆-alkylsulphonyl, phenyl, phenyloxy, phenylthio, phenylsulphonyl, benzyl, benzyloxy, benzylthio, benzylsulphonyl, phenethyl, phenylethoxy, phenylethylthio, phenylethylsulphonyl, fluorine, chlorine, bromine, cyano, trifluoromethyl, trifluoromethoxy, trifluoromethylthio, C₁-C₆-alkoxycarbonyl or by a group of the formula -NR⁵R⁶,
where
R⁵ and R⁶ have the abovementioned meaning,
X
- denotes a group of the formula -CH=CH-
and
A
- denotes a group of the formula 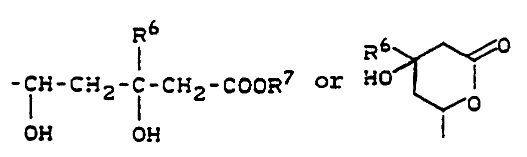 in which
R⁶
- denotes hydrogen or C₁-C₆-alkyl,
and
R⁷
- denotes C₁-C₆-alkyl, phenyl or benzyl,
or
- denotes a physiologically tolerated cation,
characterized in that
ketones of the general formula (VIII) 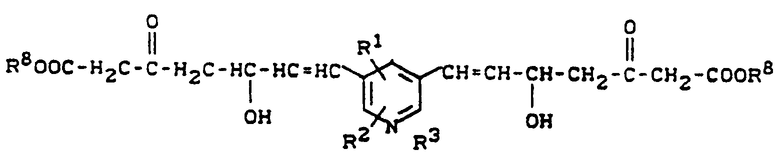 in which
R¹, R² and R³ have the abovementioned meaning,
and
R⁸
- represents alkyl,
are reduced,
the esters are hydrolysed when preparing the acids,
the carboxylic acids are cyclized when preparing the lactones,
either the esters or the lactones are hydrolysed when preparing the salts,
and isomers are optionally resolved.

2. Process for preparing ketones of the formula 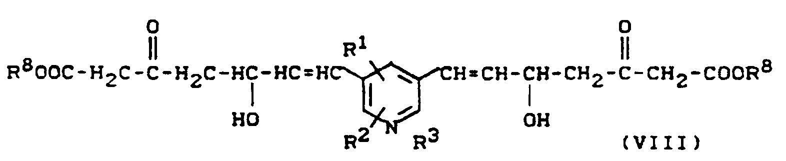 where
R¹ - R³ have the meaning given in Claim 1, and
R⁸ represents alkyl,
characterized in that
aldehydes of the general formula (IX) 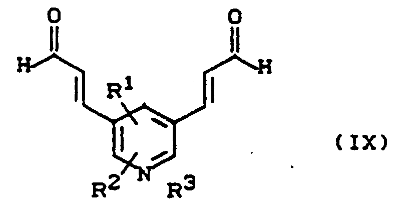 in which
R¹, R² and R³ have the abovementioned meaning,
are reacted in inert solvents with acetoacetic esters of the general formula (X) 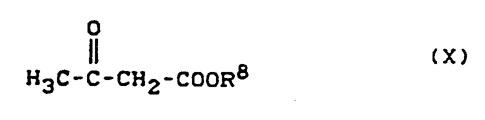 in which
R⁸ has the abovementioned meaning,
in the presence of bases.

3. Use of disubstituted pyridines of the general formulae I a and b 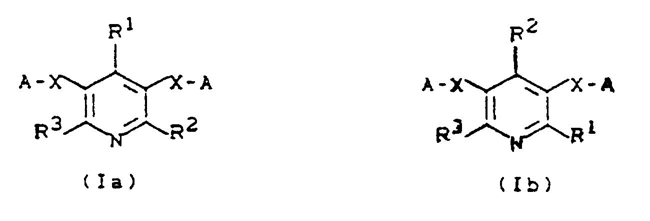 in which
R¹ - R³, A and X have the meaning given in Claim 1, for preparing medicaments.

## Revendications (Revendications pour l'(les) Etat(s) contractant(s) suivant(s): AT, BE, CH, DE, FR, GB, GR, IT, LI, NL, SE)

1. Pyridines disubstituées répondant aux formules 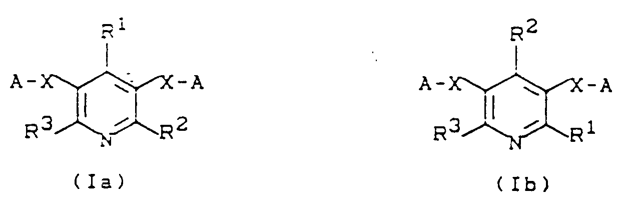 dans lesquelles
R¹ représente un groupe thiényle, un groupe furyle, un groupe thiazolyle, un groupe isothiazolyle, un groupe oxazolyle, un groupe isoxazolyle, un groupe pyridyle, un groupe pyrimidyle, un groupe pyrazinyle, un groupe pyridazinyle, un groupe indolyle, un groupe isoindolyle, un groupe quinoléyle, un groupe isoquinoléyle, un groupe phtalazinyle, un groupe quinoxalinyle, un groupe quinazolinyle, un groupe cinnolinyle, un groupe benzothiazolyle, un groupe benzoxazolyle ou un groupe benzimidazolyle, les groupes qui précèdent pouvant être substitués jusqu'à 2 fois de manière identique ou différente par un atome de fluor, par un atome de chlore, par un atome de brome, par un groupe alkyle en C₁-C₆, par un groupe alcoxy en C₁-C₆, par un groupe phényle, par un groupe phénoxy, par un groupe trifluorométhyle, par un groupe trifluorométhoxy ou par un groupe alcoxy(en C₁-C₆)carbonyle, ou représente
un groupe phényle ou un groupe naphtyle, les groupes qui précèdent pouvant être substitués jusqu'à 4 fois de manière identique ou différente par un groupe alkyle en C₁-C₆, par un groupe alcoxy en C₁-C₆, par un groupe alkyl(en C₁-C₆)thio, par un groupe alkyl(en C₁-C₆)sulfonyle, par un groupe phényle, par un groupe phényloxy, par un groupe phénylthio, par un groupe phénylsulfonyle, par un groupe benzyle, par un groupe benzyloxy, par un groupe benzylthio, par un groupe benzylsulfonyle, par un groupe phénéthyle, par un groupe phényléthoxy, par un groupe phényléthylthio, par un groupe phényléthylsulfonyle, par un atome de fluor, par un atome de chlore, par un atome de brome, par un groupe cyano, par un groupe trifluorométhyle, par un groupe trifluorométhoxy, par un groupe trifluorométhylthio, par un groupe alcoxy(en C₁-C₆)carbonyle ou encore par un groupe de formule -NR⁴R⁵
dans laquelle
R⁴ et R⁵ sont identiques ou différents et représentent un groupe alkyle en C₁-C₆, un groupe phényle, un groupe benzyle, un groupe acétyle, un groupe benzoyle, un groupe phénylsulfonyle ou un groupe alkyl(en C₁-C₆)sulfonyle,
R² représente un groupe cyclopropyle, un groupe cyclopentyle ou un groupe cyclohexyle, ou
représente un groupe alkyle en C₁-C₆ qui peut être substitué par un atome de fluor, par un atome de chlore, par un atome de brome, par un groupe cyano, par un groupe alcoxy en C₁-C₆, par un groupe alkyl(en C₁-C₆)thio, par un groupe alkyl(en C₁-C₆)sulfonyle, par un groupe phényle, par un groupe trifluorométhyle, par un groupe trifluorométhoxy, par un groupe trifluorométhylsulfonyle, par un groupe alcoxy(en C₁-C₆)carbonyle, par un groupe benzoyle, par un groupe alkyl(en C₁-C₆)carbonyle, par un groupe de formule -NR⁴R⁵
dans laquelle
R⁴ et R⁵ ont la signification indiquée ci-dessus,
ou encore par un groupe pyridyle, par un groupe pyrimidyle, par un groupe pyrazinyle, par un groupe pyridazinyle, par un groupe quinoléyle, par un groupe isoquinoléyle, par un groupe pyrrolyle, par un groupe indolyle, par un groupe thiényle, par un groupe furyle, par un groupe imidazolyle, par un groupe oxazolyle, par un groupe thiazolyle, par un groupe phényle, par un groupe phénoxy, par un groupe phénylthio, par un groupe phénylsulfonyle, par un groupe benzyloxy, par un groupe benzylthio, par un groupe benzylsulfonyle, par un groupe phényléthoxy, par un groupe phényléthylthio ou par un groupe phényléthylsulfonyle, les radicaux hétéro-aryle et aryle mentionnés pouvant être substitués jusqu'à 2 fois de manière identique ou différente par un atome de fluor, par un atome de chlore, par un atome de brome, par un groupe alkyle en C₁-C₆, par un groupe alcoxy en C₁-C₆, par un groupe trifluorométhyle ou par un groupe trifluorométhoxy,
R³ représente un atome d'hydrogène, ou
représente un groupe cyclopropyle, un groupe cyclopentyle ou un groupe cyclohexyle, ou
représente un groupe alkyle en C₁-C₆ qui peut être substitué par un atome de fluor, par un atome de chlore, par un atome de brome, par un groupe cyano, par un groupe alcoxy en C₁-C₆, par un groupe alkyl(en C₁-C₆)thio, par un groupe alkyl(en C₁-C₆)sulfonyle, par un groupe trifluorométhyle, par un groupe trifluorométhoxy, par un groupe trifluorométhylsulfonyle, par un groupe alcoxy(en C₁-C₆)carbonyle, par un groupe benzoyle, par un groupe alkyl(en C₁-C₆)carbonyle, par un groupe de formule -NR⁴R⁵
dans laquelle
R⁴ et R⁵ ont la signification indiquée ci-dessus,
ou encore par un groupe pyridyle, par un groupe pyrimidyle, par un groupe pyrazinyle, par un groupe pyridazinyle, par un groupe quinoléyle, par un groupe isoquinoléyle, par un groupe pyrrolyle, par un groupe indolyle, par un groupe thiényle, par un groupe furyle, par un groupe imidazolyle, par un groupe oxazolyle, par un groupe thiazolyle, par un groupe phényle, par un groupe phénoxy, par un groupe phénylthio, par un groupe phénylsulfonyle, par un groupe benzyloxy, par un groupe benzylthio, par un groupe benzylsulfonyle, par un groupe phényléthoxy, par un groupe phényléthylthio ou par un groupe phényléthylsulfonyle, les radicaux hétéro-aryle et aryle mentionnés pouvant être substitués jusqu'à 2 fois de manière identique ou différente par un atome de fluor, par un atome de chlore, par un atome de brome, par un groupe alkyle en C₁-C₆, par un groupe alcoxy en C₁-C₆, par un groupe trifluorométhyle ou par un groupe trifluorométhoxy,
représente un groupe thiényle, un groupe furyle, un groupe thiazolyle, un groupe isothiazolyle, un groupe oxazolyle, un groupe isoxazolyle, un groupe pyridyle, un groupe pyrimidyle, un groupe pyrazinyle, un groupe pyridazinyle, un groupe indolyle, un groupe isoindolyle, un groupe quinoléyle, un groupe isoquinoléyle, un groupe phtalazinyle, un groupe quinoxalinyle, un groupe quinazolinyle, un groupe cinnolinyle, un groupe benzothiazolyle, un groupe benzoxazolyle ou un groupe benzimidazolyle, les groupes qui précèdent pouvant être substitués jusqu'à 2 fois de manière identique ou différente par un atome de fluor, par un atome de chlore, par un atome de brome, par un groupe alkyle en C₁-C₆, par un groupe alcoxy en C₁-C₆, par un groupe phényle, par un groupe phénoxy, par un groupe trifluorométhyle, par un groupe trifluorométhoxy ou par un groupe alcoxy(en C₁-C₆)carbonyle, ou représente
un groupe phényle ou un groupe naphtyle, ces groupes pouvant être substitués jusqu'à 4 fois de manière identique ou différente par un groupe alkyle en C₁-C₆, par un groupe alcoxy en C₁-C₆, par un groupe alkyl(en C₁-C₆)thio, par un groupe alkyl(en C₁-C₆)sulfonyle, par un groupe phényle, par un groupe phényloxy, par un groupe phénylthio, par un groupe phénylsulfonyle, par un groupe benzyle, par un groupe benzyloxy, par un groupe benzylthio, par un groupe benzylsulfonyle, par un groupe phénéthyle, par un groupe phényléthoxy, par un groupe phényléthylthio, par un groupe phényléthylsulfonyle, par un atome de fluor, par un atome de chlore, par un atome de brome, par un groupe cyano, par un groupe trifluorométhyle, par un groupe trifluorométhoxy, par un groupe trifluorométhylthio, par un groupe alcoxy(en C₁-C₆)carbonyle ou par un groupe de formule -NR⁵R⁶
dans laquelle
R⁵ et R⁶ ont la signification indiquée ci-dessus,
X représente un groupe de formule -CH=CH-
et
A représente un groupe de formules 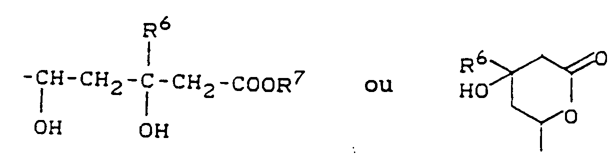 dans lesquelles
R⁶ représente un atome d'hydrogène ou un groupe alkyle en C₁-C₆,
et
R⁷ représente un groupe alkyle en C₁-C₆, un groupe phényle ou un groupe benzyle,
ou
représente un cation physiologiquement acceptable.

2. Pyridines disubstituées selon la revendication 1,
dans lesquelles
R¹ représente un groupe pyridyle, un groupe pyrimidyle, un groupe quinoléyle ou un groupe isoquinoléyle, les groupes qui précèdent pouvant être substitués par un atome de fluor, par un atome de chlore, par un groupe méthyle, par un groupe méthoxy ou encore par un groupe trifluorométhyle, ou
représente un groupe phényle qui peut être substitué jusqu'à 3 fois de manière identique ou différente par un groupe méthyle, par un groupe éthyle, par un groupe propyle, par un groupe isopropyle, par un groupe butyle, par un groupe isobutyle, par un groupe tert.-butyle, par un groupe méthoxy, par un groupe éthoxy, par un groupe propoxy, par un groupe isopropoxy, par un groupe butoxy, par un groupe isobutoxy, par un groupe tert.-butoxy, par un groupe méthylthio, par un groupe éthylthio, par un groupe propylthio, par un groupe isopropylthio, par un groupe méthylsulfonyle, par un groupe éthylsulfonyle, par un groupe propylsulfonyle, par un groupe isopropylsulfonyle, par un groupe phényle, par un groupe phénoxy, par un groupe benzyle, par un groupe benzyloxy, par un atome de fluor, par un atome de chlore, par un atome de brome, par un groupe cyano, par un groupe trifluorométhyle, par un groupe trifluorométhoxy, par un groupe méthoxycarbonyle, par un groupe éthoxycarbonyle, par un groupe propoxycarbonyle, par un groupe isopropoxycarbonyle, par un groupe butoxycarbonyle, par un groupe isobutoxycarbonyle ou par un groupe tert.-butoxycarbonyle,
R² représente un groupe cyclopropyle, un groupe cyclopentyle ou un groupe cyclohexyle, ou
représente un groupe méthyle, un groupe éthyle, un groupe propyle, un groupe isopropyle, un groupe butyle, un groupe sec.-butyle ou un groupe tert.-butyle, les groupes qui précèdent pouvant être substitués par un atome de fluor, par un atome de chlore, par un atome de brome, par un groupe cyano, par un groupe méthoxy, par groupe éthoxy, par un groupe propoxy, par un groupe isopropoxy, par un groupe butoxy, par un groupe sec.-butoxy, par un groupe tert.-butoxy, par un groupe méthylthio, par un groupe éthylthio, par un groupe propylthio, par un groupe isopropylthio, par un groupe méthylsulfonyle, par un groupe éthylsulfonyle, par un groupe propylsulfonyle, par un groupe isopropylsulfonyle, par un groupe trifluorométhyle, par un groupe trifluorométhoxy, par un groupe méthoxycarbonyle, par un groupe éthoxycarbonyle, par un groupe butoxycarbonyle, par un groupe isobutoxycarbonyle, par un groupe tert.-butoxycarbonyle, par un groupe benzoyle, par un groupe acétyle, par un groupe pyridyle, par un groupe pyrimidyle, par un groupe thiényle, par un groupe furyle, par un groupe phényle, par un groupe phénoxy, par un groupe phénylthio, par un groupe phénylsulfonyle, par un groupe benzyloxy, par un groupe benzylthio ou par un groupe benzylsulfonyle,
R³ représente un atome d'hydrogène, un groupe cyclopropyle, un groupe cyclopentyle ou un groupe cyclohexyle, ou
représente un groupe méthyle, un groupe éthyle, un groupe propyle, un groupe isopropyle, un groupe butyle, un groupe isobutyle, un groupe tert.-butyle, un groupe pentyle, un groupe isopentyle, un groupe hexyle ou un groupe isohexyle, les groupes qui précèdent pouvant être substitués par un atome de fluor, par un atome de chlore, par un atome de brome, par un groupe cyano, par un groupe méthoxy, par groupe éthoxy, par un groupe propoxy, par un groupe isopropoxy, par un groupe butoxy, par un groupe isobutoxy, par un groupe tert.-butoxy, par un groupe méthylthio, par un groupe éthylthio, par un groupe propylthio, par un groupe isopropylthio, par un groupe butylthio, par un groupe isobutylthio, par un groupe tert.-butylthio, par un groupe méthylsulfonyle, par un groupe éthylsulfonyle, par un groupe propylsulfonyle, par un groupe isopropylsulfonyle, par un groupe butylsulfonyle, par un groupe isobutylsulfonyle, par un groupe tert.-butylsulfonyle, par un groupe trifluorométhyle, par un groupe trifluorométhoxy, par un groupe méthoxycarbonyle, par un groupe éthoxycarbonyle, par un groupe propoxycarbonyle, par un groupe isopropoxycarbonyle, par un groupe butoxycarbonyle, par un groupe isobutoxycarbonyle, par un groupe tert.-butoxycarbonyle, par un groupe benzoyle, par un groupe acétyle, par un groupe éthylcarbonyle ou encore par un groupe -NR⁴R⁵
dans lequel
R⁴ et R⁵ sont identiques ou différents et représentent un groupe méthyle, un groupe éthyle, un groupe propyle, un groupe isopropyle, un groupe butyle, un groupe isobutyle, un groupe tert.-butyle, un groupe phényle, un groupe benzyle, un groupe acétyle, un groupe méthylsulfonyle, un groupe éthylsulfonyle, un groupe propylsulfonyle, un groupe isopropylsulfonyle ou un groupe phénylsulfonyle,
ou encore par un groupe pyridyle, par un groupe pyrimidyle, par un groupe pyrazinyle, par un groupe pyridazinyle, par un groupe quinoléyle, par un groupe isoquinoléyle, par un groupe thiényle, par un groupe furyle, par un groupe phényle, par un groupe phénoxy, par un groupe phénylthio, par un groupe phénylsulfonyle, par un groupe benzyloxy, par un groupe benzylthio ou par un groupe benzylsulfonyle, les radicaux hétéro-aryle et aryle mentionnés pouvant être substitués par un atome de fluor, par un atome de chlore, par un groupe méthyle, par un groupe éthyle, par un groupe propyle, par un groupe isopropyle, par un groupe isobutyle, par un groupe tert.-butyle, par un groupe méthoxy, par un groupe éthoxy, par un groupe propoxy, par un groupe isopropoxy, par un groupe butoxy, par un groupe isobutoxy, par un groupe tert.-butoxy, par un groupe trifluorométhyle ou par un groupe trifluorométhoxy, ou
représente un groupe thiényle, un groupe furyle, un groupe pyridyle, un groupe pyrimidyle, un groupe pyrazinyle, un groupe pyridazinyle, un groupe oxazolyle, un groupe isooxazolyle, un groupe imidazolyle, un groupe pyrazolyle, un groupe thiazolyle, un groupe isothiazolyle, un groupe quinoléyle, un groupe isoquinoléyle, un groupe benzoxazolyle, un groupe benzimidazolyle ou un groupe benzothiazolyle, Les radicaux mentionnés pouvant être substitués par un atome de fluor, par un atome de chlore, par un groupe méthyle, par un groupe éthyle, par un groupe propyle, par un groupe isopropyle, par un groupe butyle, par un groupe isobutyle, par un groupe tert.-butyle, par un groupe méthoxy, par un groupe éthoxy, par un groupe propoxy, par un groupe isopropoxy, par un groupe butoxy, par un groupe isobutoxy, par un groupe tert.-butoxy, par un groupe phényle, par un groupe phénoxy, par un groupe trifluorométhyle, par un groupe trifluorométhoxy, par un groupe méthoxycarbonyle, par un groupe éthoxycarbonyle, par un groupe isopropoxycarbonyle, par un groupe propoxycarbonyle, par un groupe butoxycarbonyle, par un groupe isobutoxycarbonyle ou par un groupe tert.-butoxycarbonyle, ou
représente un groupe phényle qui peut être substitué jusqu'à 3 fois de manière identique ou différente par un groupe méthyle, par un groupe éthyle, par un groupe propyle, par un groupe isopropyle, par un groupe butyle, par un groupe isobutyle, par un groupe tert.-butyle, par un groupe pentyle, par un groupe isopentyle, par un groupe hexyle, par un groupe isohexyle, par un groupe méthoxy, par un groupe éthoxy, par un groupe propoxy, par un groupe isopropoxy, par un groupe butoxy, par un groupe isobutoxy, par un groupe tert.-butoxy, par un groupe méthylthio, par un groupe éthylthio, par un groupe propylthio, par un groupe isopropylthio, par un groupe butylthio, par un groupe isobutylthio, par un groupe tert.-butylthio, par un groupe méthylsulfonyle, par un groupe éthylsulfonyle, par un groupe propylsulfonyle, par un groupe isopropylsulfonyle, par un groupe butylsulfonyle, par un groupe isobutylsulfonyle, par un groupe tert.-butylsulfonyle, par un groupe phényle, par un groupe phénoxy, par un groupe phénylthio, par un groupe phénylsulfonyle, par un groupe benzyle, par un groupe benzyloxy, par un groupe benzylthio, par un groupe benzylsulfonyle, par un atome de fluor, par un atome de chlore, par un atome de brome, par un groupe cyano, par un groupe trifluorométhyle, par un groupe trifluorométhoxy, par un groupe trifluorométhylthio, par un groupe méthoxycarbonyle, par un groupe éthoxycarbonyle, par un groupe propoxycarbonyle, par un groupe isopropoxycarbonyle, par un groupe butoxycarbonyle, par un groupe isobutoxycarbonyle, par un groupe tert.-butoxycarbonyle ou encore par un groupe -NR⁴R⁵
dans lequel
R⁴ et R⁵ ont la signification indiquée ci-dessus,
X représente un groupe de formule -CH=CH-
et
A représente un groupe de formules 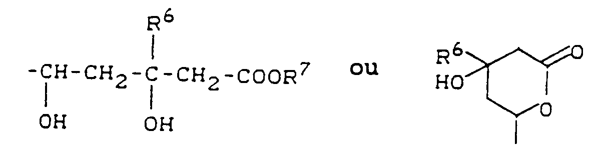 dans lesquelles
R⁶ représente un atome d'hydrogène, un groupe méthyle, un groupe éthyle, un groupe propyle, un groupe isopropyle, un groupe butyle, un groupe isobutyle ou un groupe tert.-butyle,
et
R⁷ représente un atome d'hydrogène, un groupe méthyle, un groupe éthyle, un groupe propyle, un groupe isopropyle, un groupe butyle, un groupe isobutyle, un groupe tert.-butyle ou un groupe benzyle,
ou représente un ion sodium, potassium, calcium ou magnésium, ou encore un ion ammonium.

3. Pyridines disubstituées selon la revendication 1,
dans lesquelles
R¹ représente un groupe phényle qui peut être substitué jusqu'à 2 fois de manière identique ou différente par un groupe méthyle, par un groupe propyle, par un groupe isopropyle, par un groupe phénoxy et/ou par un atome de fluor,
R² représente un groupe cyclopropyle ou un groupe cyclohexyle ou encore un groupe méthyle, un groupe éthyle, un groupe propyle, un groupe isopropyle, un groupe butyle, un groupe isobutyle ou un groupe tert.-butyle, les groupes qui précèdent pouvant être substitués par un atome de fluor, par un atome de chlore, par un groupe méthoxy, par un groupe phényle ou par un groupe phénoxy,
R³ représente un atome d'hydrogène, un groupe cyclopropyle, un groupe cyclopentyle ou un groupe cyclohexyle ou encore un groupe phényle, ou
représente un groupe méthyle, un groupe éthyle, un groupe propyle, un groupe isopropyle, un groupe butyle ou un groupe benzyle,
X représente un groupe de formule 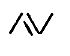 (à configuration E)
et
A représente un groupe de formules 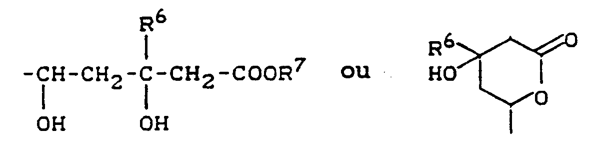 dans lesquelles
R⁶ représente un atome d'hydrogène,
et
R⁷ représente un atome d'hydrogène, un groupe méthyle ou un groupe éthyle,
ou
représente un ion sodium ou potassium.

4. Pyridines disubstituées selon les revendications 1 à 3 à des fins de traitement thérapeutique.

5. Procédé pour la préparation de pyridines disubstituées selon la revendication 1, caractérisé en ce qu'on réduit des cétones répondant à la formule générale (VIII) 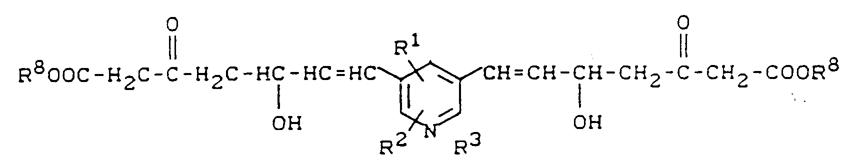 dans laquelle
R¹, R² et R³ ont la signification indiquée ci-dessus,
et
R⁸ représente un groupe alkyle en C₁-C₁₂,
dans le cas de la préparation des acides, on saponifie les esters,
dans le cas de la préparation des lactones, on cyclise les acides carboxyliques,
dans le cas de la préparation des sels, on saponifie, soit les esters, soit les lactones,
et éventuellement, on sépare les isomères.

6. Cétones répondant à la formule 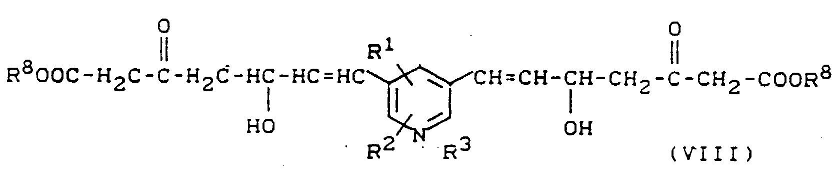 dans laquelle
R¹, R² et R³ ont la signification indiquée à la revendication 1,
et
R⁸ représente un groupe alkyle en C₁-C₁₂.

7. Procédé selon la revendication 8, caractérisé en ce qu'on effectue la réaction dans un domaine de température de -80°C à +50°C.

8. Médicament contenant des pyridines disubstituées selon les revendications 1 à 3.

9. Utilisation de pyridines disubstituées selon les revendications 1 à 3, pour la préparation de médicaments.

10. Utilisation selon la revendication 9, pour la préparation de médicaments pour le traitement de la lipoprotéinémie et de l'artériosclérose.

11. Médicament selon la revendication 10, contenant de 0,5 à 98,5% en poids de pyridines disubstituées.

## Revendications (Revendications pour l'(les) Etat(s) contractant(s) suivant(s): ES)

1. Procédé pour la préparation de pyridines disubstituées répondant aux formules 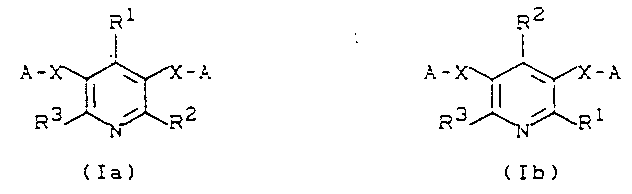 dans lesquelles
R¹ représente un groupe thiényle, un groupe furyle, un groupe thiazolyle, un groupe isothiazolyle, un groupe oxazolyle, un groupe isoxazolyle, un groupe pyridyle, un groupe pyrimidyle, un groupe pyrazinyle, un groupe pyridazinyle, un groupe indolyle, un groupe isoindolyle, un groupe quinoléyle, un groupe isoquinoléyle, un groupe phtalazinyle, un groupe quinoxalinyle, un groupe quinazolinyle, un groupe cinnolinyle, un groupe benzothiazolyle, un groupe benzoxazolyle ou un groupe benzimidazolyle, les groupes qui précèdent pouvant être substitués jusqu'à 2 fois de manière identique ou différente par un atome de fluor, par un atome de chlore, par un atome de brome, par un groupe alkyle en C₁-C₆, par un groupe alcoxy en C₁-C₆, par un groupe phényle, par un groupe phénoxy, par un groupe trifluorométhyle, par un groupe trifluorométhoxy ou par un groupe alcoxy(en C₁-C₆)carbonyle, ou représente
un groupe phényle ou un groupe naphtyle, ces groupes pouvant être substitués jusqu'à 4 fois de manière identique ou différente par un groupe alkyle en C₁-C₆, par un groupe alcoxy en C₁-C₆, par un groupe alkyl(en C₁-C₆)thio, par un groupe alkyl(en C₁-C₆)sulfonyle, par un groupe phényle, par un groupe phényloxy, par un groupe phénylthio, par un groupe phénylsulfonyle, par un groupe benzyle, par un groupe benzyloxy, par un groupe benzylthio, par un groupe benzylsulfonyle, par un groupe phénéthyle, par un groupe phényléthoxy, par un groupe phényléthylthio, par un groupe phényléthylsulfonyle, par un atome de fluor, par un atome de chlore, par un atome de brome, par un groupe cyano, par un groupe trifluorométhyle, par un groupe trifluorométhoxy, par un groupe trifluorométhylthio, par un groupe alcoxy(en C₁-C₆)carbonyle ou encore par un groupe de formule -NR⁴R⁵
dans laquelle
R⁴ et R⁵ sont identiques ou différents et représentent un groupe alkyle en C₁-C₆, un groupe phényle, un groupe benzyle, un groupe acétyle, un groupe benzoyle, un groupe phénylsulfonyle ou un groupe alkyl(en C₁-C₆)sulfonyle,
R² représente un groupe cyclopropyle, un groupe cyclopentyle ou un groupe cyclohexyle, ou
représente un groupe alkyle en C₁-C₆ qui peut être substitué par un atome de fluor, par un atome de chlore, par un atome de brome, par un groupe cyano, par un groupe alcoxy en C₁-C₆, par un groupe alkyl(en C₁-C₆)thio, par un groupe alkyl(en C₁-C₆)sulfonyle, par un groupe phényle, par un groupe trifluorométhyle, par un groupe trifluorométhoxy, par un groupe trifluorométhylsulfonyle, par un groupe alcoxy(en C₁-C₆)carbonyle, par un groupe benzoyle, par un groupe alkyl(en C₁-C₆)carbonyle, par un groupe de formule -NR⁴R⁵
dans laquelle
R⁴ et R⁵ ont la signification indiquée ci-dessus,
ou encore par un groupe pyridyle, par un groupe pyrimidyle, par un groupe pyrazinyle, par un groupe pyridazinyle, par un groupe quinoléyle, par un groupe isoquinoléyle, par un groupe pyrrolyle, par un groupe indolyle, par un groupe thiényle, par un groupe furyle, par un groupe imidazolyle, par un groupe oxazolyle, par un groupe thiazolyle, par un groupe phényle, par un groupe phénoxy, par un groupe phénylthio, par un groupe phénylsulfonyle, par un groupe benzyloxy, par un groupe benzylthio, par un groupe benzylsulfonyle, par un groupe phényléthoxy, par un groupe phényléthylthio ou par un groupe phényléthylsulfonyle, les radicaux hétéro-aryle et aryle mentionnés pouvant être substitués jusqu'à 2 fois de manière identique ou différente par un atome de fluor, par un atome de chlore, par un atome de brome, par un groupe alkyle en C₁-C₆, par un groupe alcoxy en C₁-C₆, par un groupe trifluorométhyle ou par un groupe trifluorométhoxy,
R³ représente un atome d'hydrogène, ou
représente un groupe cyclopropyle, un groupe cyclopentyle ou un groupe cyclohexyle, ou
représente un groupe alkyle en C₁-C₆ qui peut être substitué par un atome de fluor, par un atome de chlore, par un atome de brome, par un groupe cyano, par un groupe alcoxy en C₁-C₆, par un groupe alkyl(en C₁-C₆)thio, par un groupe alkyl(en C₁-C₆)sulfonyle, par un groupe trifluorométhyle, par un groupe trifluorométhoxy, par un groupe trifluorométhylsulfonyle, par un groupe alcoxy(en C₁-C₆)carbonyle, par un groupe benzoyle, par un groupe alkyl(en C₁-C₆)carbonyle, par un groupe de formule -NR⁴R⁵
dans laquelle
R⁴ et R⁵ ont la signification indiquée ci-dessus,
ou encore par un groupe pyridyle, par un groupe pyrimidyle, par un groupe pyrazinyle, par un groupe pyridazinyle, par un groupe quinoléyle, par un groupe isoquinoléyle, par un groupe pyrrolyle, par un groupe indolyle, par un groupe thiényle, par un groupe furyle, par un groupe imidazolyle, par un groupe oxazolyle, par un groupe thiazolyle, par un groupe phényle, par un groupe phénoxy, par un groupe phénylthio, par un groupe phénylsulfonyle, par un groupe benzyloxy, par un groupe benzylthio, par un groupe benzylsulfonyle, par un groupe phényléthoxy, par un groupe phényléthylthio ou par un groupe phényléthylsulfonyle, les radicaux hétéro-aryle et aryle mentionnés pouvant être substitués jusqu'à 2 fois de manière identique ou différente par un atome de fluor, par un atome de chlore, par un atome de brome, par un groupe alkyle en C₁-C₆, par un groupe alcoxy en C₁-C₆, par un groupe trifluorométhyle ou par un groupe trifluorométhoxy,
représente un groupe thiényle, un groupe furyle, un groupe thiazolyle, un groupe isothiazolyle, un groupe oxazolyle, un groupe isoxazolyle, un groupe pyridyle, un groupe pyrimidyle, un groupe pyrazinyle, un groupe pyridazinyle, un groupe indolyle, un groupe isoindolyle, un groupe quinoléyle, un groupe isoquinoléyle, un groupe phtalazinyle, un groupe quinoxalinyle, un groupe quinazolinyle, un groupe cinnolinyle, un groupe benzothiazolyle, un groupe benzoxazolyle ou un groupe benzimidazolyle, les groupes qui précèdent pouvant être substitués jusqu'à 2 fois de manière identique ou différente par un atome de fluor, par un atome de chlore, par un atome de brome, par un groupe alkyle en C₁-C₆, par un groupe alcoxy en C₁-C₆, par un groupe phényle, par un groupe phénoxy, par un groupe trifluorométhyle, par un groupe trifluorométhoxy ou par un groupe alcoxy(en C₁-C₆)carbonyle, ou représente
un groupe phényle ou un groupe naphtyle, ces groupes pouvant être substitués jusqu'à 4 fois de manière identique ou différente par un groupe alkyle en C₁-C₆, par un groupe alcoxy en C₁-C₆, par un groupe alkyl(en C₁-C₆)thio, par un groupe alkyl(en C₁-C₆)sulfonyle, par un groupe phényle, par un groupe phényloxy, par un groupe phénylthio, par un groupe phénylsulfonyle, par un groupe benzyle, par un groupe benzyloxy, par un groupe benzylthio, par un groupe benzylsulfonyle, par un groupe phénéthyle, par un groupe phényléthoxy, par un groupe phényléthylthio, par un groupe phényléthylsulfonyle, par un atome de fluor, par un atome de chlore, par un atome de brome, par un groupe cyano, par un groupe trifluorométhyle, par un groupe trifluorométhoxy, par un groupe trifluorométhylthio, par un groupe alcoxy(en C₁-C₆)carbonyle ou par un groupe de formule -NR⁵R⁶
dans laquelle
R⁵ et R⁶ ont la signification indiquée ci-dessus,
X représente un groupe de formule -CH=CH-
et
A représente un groupe de formules 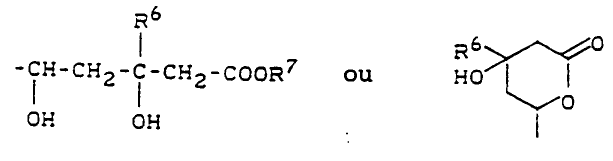 dans lesquelles
R⁶ représente un atome d'hydrogène ou un groupe alkyle en C₁-C₆,
et
R⁷ représente un groupe alkyle en C₁-C₆, un groupe phényle ou un groupe benzyle,
ou
représente un cation physiologiquement acceptable,
caractérisé en ce qu'on réduit des cétones répondant à la formule générale (VIII) 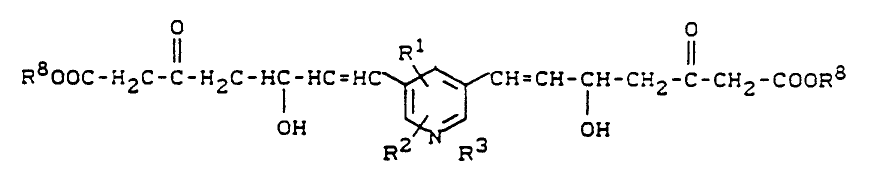 dans laquelle
R¹, R² et R³ ont la signification indiquée ci-dessus,
et
R⁸ représente un groupe alkyle,
dans le cas de la préparation des acides, on saponifie les esters,
dans le cas de la préparation des lactones, on cyclise les acides carboxyliques,
dans le cas de la préparation des sels, on saponifie, soit les esters, soit les lactones,
et éventuellement, on sépare les isomères.

2. Procédé pour la préparation de cétones de formule 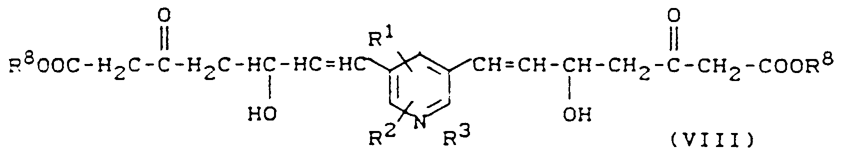 dans laquelle
R¹-R³ ont la signification indiquée à la revendication 1,
et
R⁸ représente un groupe alkyle,
caractérisé en ce qu'on fait réagir
des aldéhydes répondant à la formule générale (IX) 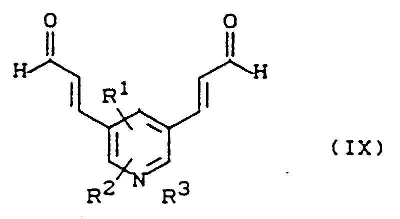 dans laquelle
R¹, R² et R³ ont la signification indiquée ci-dessus,
dans des solvants inertes avec de l'ester acétoacétique répondant à la formule générale (X) 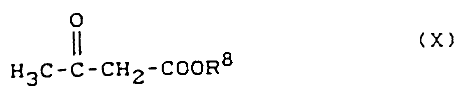 dans laquelle
R⁸ a la signification indiquée ci-dessus,
en présence de bases.

3. Utilisation de pyridines disubstituées répondant aux formules générales Ia, b 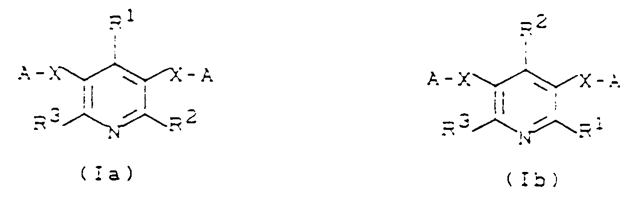 dans lesquelles
R¹-R³, A et X ont la signification indiquée à la revendication 1, pour la préparation de médicaments.
